# FASCICULE DE BREVET EUROPEEN

(11) **EP 1 042 323 B1**
(45) Date de publication et mention de la délivrance du brevet: **10.04.2002**
(21) Numéro de dépôt: 98962480.4
(22) Date de dépôt: 16.12.1998
(51) Int. Cl.: C07D 471/14, A61K 31/44, C07D 213/64

(54) **NOUVEAUX ANALOGUES TETRACYCLIQUES DE CAMPTOTHECINES, LEURS PROCEDES DE PREPARATION, LEUR APPLICATION COMME MEDICAMENTS ET LES COMPOSITIONS PHARMACEUTIQUES LES CONTENANT**
TETRACYCLISCHE CAMPOTHECINEANALOGUE, VERFAHREN ZU IHRER HERSTELLUNG, IHRE VERWENDUNG ALS ARZNEIMITTEL UND DIESE ENTHALTENDE PHARMAZEUTISCHE ZUBEREITUNGEN
NOVEL CAMPTOTHECIN TETRACYCLIC ANALOGUES, PREPARATION, METHODS, APPLICATIONS AS MEDICINES AND PHARMACEUTICAL COMPOSITIONS CONTAINING THEM

(30) Priorité: 24.12.1997 FR 9716461
(43) Date de publication de la demande: 11.10.2000
(73) Titulaire: SOCIETE DE CONSEILS DE RECHERCHES ET D'APPLICATIONS SCIENTIFIQUES (S.C.R.A.S.), 75016 Paris (FR)
(72) Inventeur: BIGG, Dennis, F-91190 Gif-sur-Yvette (FR); LAVERGNE, Olivier, F-91120 Palaiseau (FR); ROLLAND, Alain, F-91120 Palaiseau (FR); LANCO, Christophe, F-91410 Dourdan (FR); ULIBARRI, Gérard, F-91440 Bures-sur-Yvette (FR)
(74) Mandataire: Bourgouin, André
(86) Numéro de dépôt international: FR9802751
(87) Numéro de publication internationale: WO9933829

(56) Documents cités:
- WO-A-97/00876
- CHEMICAL ABSTRACTS, vol. 127, no. 25, 1997 Columbus, Ohio, US; abstract no. 346275t, R. FUJITA ET AL.: "Investigation of coupling constants and chemical shifts in 1H- and 13C-NMR spectra of 2-pyridones" page 561; XP002077294 & ANNU. REP. TOHOKU COLL. PHARM., vol. 43, 1996, pages 79-84,

## Description

La camptothécine est un composé naturel qui a été isolé pour la première fois des feuilles et de l'écorce de la plante chinoise appelée *camptotheca acuminata* (voir Wall et ses collaborateurs, J. Amer. Chem. Soc. 88:3888 (1966)). La camptothécine est un composé pentacyclique, constitué d'un fragment indolizino[1,2-b]quinoléine fusionné avec une α-hydroxylactone à six chaînons, et répond à la formule suivante :

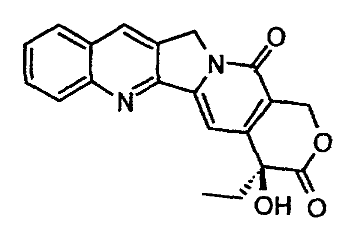

La camptothécine présente une activité anti-proliférative dans plusieurs lignées cellulaires cancéreuses, comprenant les lignées cellulaires de tumeurs humaines du colon, du poumon et du sein (Suffness, M. et collaborateurs : The Alkaloids Chemistry and Pharmacology, Bross, A., ed., Vol. 25, p. 73 (Academic Press, 1985)). Il est établi que l'activité anti-proliférative de la camptothécine est en relation avec son activité inhibitrice de la topoisomérase I de l'ADN.

Il avait été indiqué que l'α-hydroxylactone était une exigence absolue à la fois pour l'activité *in vivo* et *in vitro* de la camptothécine (Camptothecins: New Anticancer Agents, Putmesil, M., et ses collaborateurs, ed., p. 27 (CRC Press, 1995); Wall, M. et ses collaborateurs, Cancer Res. 55:753 (1995) ; Hertzberg et ses collaborateurs, J. Med. Chem. 32:715 (1982) et Crow et ses collaborateurs, J. Med. Chem. 35:4160 (1992)).

De façon inattendue, la demanderesse avait découvert que les β-hydroxylactones à 7 chaînons ont une activité biologique comparable ou supérieure à celle des α-hydroxylactones (demande PCT n° FR 96/00980 publiée sous le n° WO 97/00876). Dans ce document, la demanderesse avait décrit en tant qu'inhibiteurs de topoisomérases des composés de formules générales **(A1)** et **(A2)** 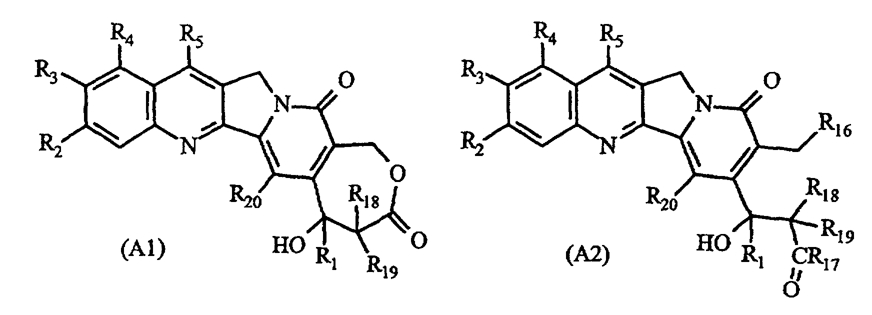 dans lesquelles :
R₁ représente notamment un radical alkyle,
R₂, R₃, R₄ et R₅ représentent notamment indépendamment un atome d'hydrogène, un atome halogène ou un radical alkyle,
R₁₆ représente notamment un atome d'hydrogène ou un radical alkoxy,
R₁₇ représente notamment un radical hydroxy, alkoxy, amino, alkylamino ou dialkylamino
et R₁₈, R₁₉ et R₂₀ représentent notamment des atomes d'hydrogène.

Or, la demanderesse vient maintenant de découvrir de façon inattendue que des analogues de camptothécine ne comprenant ni l'α-hydroxylactone ni une β-hydroxylactone présentent également une activité inhibitrice des topoisomérases. La présente invention concerne donc une nouvelle classe d'analogues tétracycliques de la camptothécine, dans lesquels l'α-hydroxylactone naturelle de la camptothécine ou la β-hydroxylactone des analogues décrits auparavant par la demanderesse est absente. Les composés selon la présente invention présentent une activité biologique puissante d'inhibition de la topoisomérase I et/ou de la topoisomérase II, ce qui est inattendu au regard de l'état de l'art antérieur.

L'invention a donc pour objet des composés de formule (I) 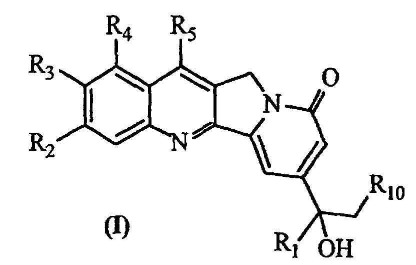 sous forme racémique, d'énantiomère ou toute combinaison de ces formes, dans laquelle
- R₁: représente un radical alkyle inférieur, alkényle inférieur, alkynyle inférieur, haloalkyle inférieur, alkoxyalkyle inférieur ou alkylthioalkyle inférieur;
- R₂, R₃ et R₄: représentent, indépendamment, un radical H, hydroxy, alkoxy inférieur, arylalkoxy, halo, haloalkyle inférieur, alkyle inférieur, alkényle inférieur, cyano, cyanoalkyle inférieur, nitro, nitroalkyle inférieur, amido, amidoalkyle inférieur, (CH₂)ₘNR₆R₇, (CH₂)ₘOR₆, (CH₂)ₘSR₆, (CH₂)ₘCO₂R₆, (CH₂)ₘNR₆C(O)R₈, (CH₂)ₘC(O)R₈, (CH₂)ₘOC(O)R₈, O(CH₂)ₘNR₆R₇, OC(O)NR₆R₇, OC(O)(CH₂)ₘCO₂R₆, aryle ou arylealkyle inférieur substitué (c'est-à-dire, substitué une à quatre fois sur le groupe aryle) ou non substitué, dans lequel le substituant est un alkyle inférieur, halo, nitro, amino, alkylamino inférieur, haloalkyle inférieur, hydroxyalkyle inférieur, alkoxy inférieur, ou alkoxyalkyle inférieur) ou R₂ et R₃, ou R₃ et R₄, ou R₄ et R₅, indépendamment forment ensemble une chaîne à 3 ou 4 chaînons, dans laquelle les éléments de la chaîne sont sélectionnés parmi le groupe constitué de CH, CH₂, O, S, N ou NR₉;
- R₅: représente un radical H, halo, haloalkyle inférieur, alkyle inférieur, alkoxy inférieur, alkoxyalkyle inférieur, alkylthioalkyle inférieur, cycloalkyle, cycloalkylalkyle inférieur, cyano, cyanoalkyle, alcanesulphonylalkyle inférieur, hydroxyalkyle inférieur, nitro, (CH₂)ₘC(O)R₈, (CH₂)ₘNR₆C(O)R₈, (CH₂)ₘNR₆R₇, (CH₂)ₘN(CH₃)(CH₂)ₙNR₆R₇, (CH₂)ₘOC(O)R₈, (CH₂)ₘOC(O)N₆R₇, aryle ou arylalkyle inférieur substitué (c'est-à-dire une à quatre fois sur le groupe aryle) ou non substitué, dans lequel le substituant est un alkyle inférieur, halo, nitro, amino, alkylamino inférieur, haloalkyle inférieur, hydroxyalkyle inférieur, alkoxy inférieur ou alkoxyalkyle inférieur,
- R₆ et R₇: représentent, indépendamment, H, un radical alkyle inférieur, hydroxyalkyle inférieur, alkylaminoalkyle inférieur, aminoalkyle inférieur, cycloalkyle, cycloalkylalkyle inférieur, alkényle inférieur, alkoxyalkyle inférieur, haloalkyle inférieur, ou aryle ou arylalkyle inférieur substitué (c'est-à-dire, une à quatre fois sur le groupe aryle) ou non substitué, dans lequel le substituant est un alkyle inférieur, halo, nitro, amino, alkylamino inférieur, haloalkyle inférieur, hydroxyalkyle inférieur, alkoxy inférieur,
ou alkoxyalkyle inférieur, ou bien, lorsque les chaînes R₆ et R₇ sont rattachées à un même atome d'azote, R₆ et R₇ forment éventuellement ensemble un hétérocycle aromatique ou non aromatique, par exemple un hétérocycle de type morpholine, pipérazine ou pipéridine, ledit hétérocycle étant éventuellement substitué par un ou plusieurs groupes choisis parmi les radicaux alkyle inférieur, aryle substitué ou non substitué, arylalkyle substitué ou non substitué, halo, nitro, amino, alkylamino inférieur, haloalkyle inférieur, hydroxyalkyle inférieur, alkoxy inférieur ou alkoxyalkyle inférieur,
- R₈: représente un radical H, alkyle inférieur, hydroxyalkyle inférieur, amino, alkylamino inférieur, alkylaminoalkyle inférieur, aminoalkyle inférieur, cycloalkyle, cycloalkylalkyle inférieur, alkényle inférieur, alkoxy inférieur, alkoxyalkyle inférieur, haloalkyle inférieur, ou aryle ou arylalkyle inférieur substitué (c'est-à-dire, une à quatre fois sur le groupe aryle) ou non substitué, dans lequel le substituant est un alkyle inférieur, halo, nitro, amino, alkylamino inférieur, haloalkyle inférieur, hydroxyalkyle inférieur, alkoxyinférieur, ou alkoxyalkyle inférieur;
- R₉: représente un radical H, alkyle inférieur, haloalkyle inférieur, aryle ou arylalkyle, le groupe aryle ou arylalkyle pouvant éventuellement être substitué sur le cycle aromatique par un ou plusieurs groupes choisis parmi le radical alkyle inférieur, halo, nitro, amino, alkylamino inférieur, haloalkyle inférieur, hydroxyalkyle inférieur, alkoxy inférieur, ou alkoxyalkyle inférieur,
- R₁₀: représente un radical cyano, C(O)OR₁₁, 1*H*-1,2,3,4-tétrazo-5-yle ou 1-alkyl-1,2,3,4-tétrazo-5-yle;
- R₁₁: représente un radical H, alkyle inférieur, haloalkyle inférieur, hydroxyalkyle inférieur, alkylcarbonyloxyalkyle, (CH₂)ₚNR₆R₇, aryle, arylalkyle ou aryle, le groupe aryle ou arylalkyle pouvant éventuellement être substitué sur le cycle aromatique par un ou plusieurs groupes choisis parmi le radical alkyle inférieur, halo, nitro, amino, alkylamino inférieur, haloalkyle inférieur, hydroxyalkyle inférieur, alkoxy inférieur, ou alkoxyalkyle inférieur;
- m: est un nombre entier compris entre 0 et 6;
- n: est un nombre entier compris entre 1 et 4;
- p: est un nombre entier compris entre 2 et 6;
ou un sel pharmaceutiquement acceptable de ce dernier.

Lorsqu'il est utilisé sans plus de précision, le terme alkyl(e) fait référence à un radical alkyle inférieur. Tel qu'il est utilisé dans ce texte, le terme inférieur en référence aux groupes alkyle, alkylthio et alkoxy désigne des groupes hydrocarbonés aliphatiques saturés, linéaires, ou ramifiés, comportant de 1 à 6 atomes de carbone, comme par exemple méthyle, éthyle, propyle, isopropyle, butyle, t-butyle, méthylthio, éthylthio, méthoxy et éthoxy. En référence aux groupes alkényle ou alkynyle, le terme inférieur désigne des groupes comportant 2 à 6 atomes de carbone et une ou plusieurs doubles ou triples liaisons, comme par exemple les groupes vinyle, allyle, isopropényle, pentènyle, hexanyle, propènyle éthynyle, propynyle et butynyle. Le terme cycloalkyle désigne un cycle de 3 à 7 carbones, comme par exemple les groupes cyclopropyle, cyclobutyle, cyclopentyle ou cyclohexyle. Le terme aryle désigne un composé hydrocarboné mono-, di ou tricyclique avec au moins un cycle aromatique, chaque cycle contenant au maximum 7 chaînons, comme par exemple phényle, naphtyle, anthracyle, biphényle ou indényle . Le terme halo signifie chloro, bromo, iodo ou fluoro. Les radicaux correspondant aux expressions haloalkyle inférieur, cyanoalkyle inférieur, nitroalkyle inférieur, amidoalkyle inférieur, hydrazinoalkyle inférieur, azidoalkyle inférieur, arylalkyle inférieur, hydroxyalkyle inférieur, alkoxyalkyle inférieur, alkylthioalkyle inférieur, et alkanesulphonylalkyle inférieur sont substitués, respectivement, par un à trois groupes halo, cyano, nitro, amido, hydrazino, azido, aryle, hydroxy, alkoxy inférieur, alkylthio inférieur ou sulphonyle inférieur. Le radical alkylamino inférieur peut contenir un ou deux groupes alkyle inférieur, et représenter par exemple NHCH₃, NHCH₂CH₃, N(CH₃)₂, ou N(CH₃)(CH₂CH₃).

Les composés selon la présente invention portent un carbone asymétrique. Par conséquent, les composés selon la présente invention ont deux formes énantiomères possibles, c'est-à-dire sous les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

De préférence, l'invention a pour objet des composés de formule (I) telle que définie ci-dessus dans laquelle R₁ représente un radical alkyle inférieur, R₂ représente un atome d'hydrogène ou d'halogène, R₃ représente un atome d'hydrogène ou d'halogène, un radical alkyle inférieur ou alkoxy inférieur, R₄ représente un atome d'hydrogène, R₅ représente un atome d'hydrogène ou un radical alkyle inférieur, R₁₀ représente un radical cyano, C(O)OR₁₁ ou 1H-1,2,3,4-tétrazo-5-yle, et enfin R₁₁ représente un radical H, alkyle inférieur, haloalkyle inférieur, hydroxyalkyle inférieur, alkylcarbonyloxyalkyle, (CH₂)ₚNR₆R₇, aryle, arylalkyle ou aryle, le groupe aryle ou arylalkyle pouvant éventuellement être substitué sur le cycle aromatique par un ou plusieurs groupes choisis parmi le radical alkyle inférieur, halo, nitro, amino, alkylamino inférieur, haloalkyle inférieur, hydroxyalkyle inférieur, alkoxy inférieur, ou alkoxyalkyle inférieur; ou un sel pharmaceutiquement acceptable de ces composés.

Selon une autre variante préférée de l'invention, R₁ représente un groupe éthyle, R₂ représente un atome d'hydrogène ou de chlore ou de fluor, R₃ représente H, un radical alkyle inférieur, halo, ou OR₆ dans lequel R₆ représente H, un radical alkyle inférieur ou arylalkyle inférieur, et de préférence H, fluoro, chloro, méthyle, méthoxy ou benzyloxy, R₄ représente un atome d'hydrogène, R₅ représente un atome d'hydrogène ou un radical alkyle inférieur, R₁₀ représente un radical cyano, C(O)OR₁₁ ou 1H-1,2,3,4-téuazo-5-yle, et enfin R₁₁ représente un radical H, alkyle inférieur, haloalkyle inférieur, hydroxyalkyle inférieur, alkylcarbonyloxyalkyle, (CH₂)ₚNR₆R₇, aryle, arylalkyle ou aryle, le groupe aryle ou arylalkyle pouvant éventuellement être substitué sur le cycle aromatique par un ou plusieurs groupes choisis parmi le radical alkyle inférieur, halo, nitro, amino, alkylamino inférieur, haloalkyle inférieur, hydroxyalkyle inférieur, alkoxy inférieur, ou alkoxyalkyle inférieur.

Plus particulièrement, l'invention a pour objet les produits décrits ci-après dans les exemples, et répondant aux formules suivantes :
- le 3-(9-benzyloxy-10-fluoro-4-oxo-4,6-dihydroindolizino [1,2-*b*]quinoléin-2-yl)-3-hydroxypentanoate de *tert*-butyle;
- le 3-(10-fluoro-9-méthoxy-4-oxo-4,6-dihydroindolizino [1,2-*b*] quinoléin-2-yl)-3-hydroxypentanoate de *tert*-butyle;
- le 3-hydroxy-3-(7-méthyl-4-oxo-4,6-dihydroindolizino [1,2-*b*] quinoléin-2-yl)pentanoate de *tert*-butyle;
- le 3-hydroxy-3-(4-oxo-4,6-dihydroindolizino [1,2-*b*] quinoléin-2-yl)pentanitrile;
- le 3-hydroxy-3-(4-oxo-4,6-dihydroindolizino [1,2-*b*] quinoléin-2-yl)pentanoate de *tert*-butyle;
- l'acide 3-hydroxy-3-(4-oxo-4,6-dihydroindolizino [1,2-*b*] qiunoléin-2-yl)pentanoïque;
- l'acide 3-(9-benzyloxy-10-fluoro-4-oxo-4,6-dihydroindolizino [1,2-*b*]quinoléin-2-yl)-3-hydroxypentanoïque;
- l'acide 3-(10-fluoro-9-méthoxy-4-oxo-4,6-dihydro-indolizino [1,2-*b*] quinoléin-2-yl)-3-hydroxypentanoïque;
- l'acide 3-hydroxy-3-(7-méthyl-4-oxo-4,6-dihydroindolizino [1,2-*b*] quinoléin-2-yl)pentanoïque;
- l'acide 3-(9-benzyloxy-4-oxo-4,6-dihydroindolizino [1,2-*b*] quinoléin-2-yl)-3-hydroxypentanoique;
- l'acide 3-(10-chloro-9-méthyl-4-oxo-4,6-dihydro-indolizino [1,2-*b*] quinoléin-2-yl)-3-hydroxypentanoïque;
- le 3-hydroxy-3-(4-oxo-4,6-dihydroindolizino [1,2-*b*] quinoléin-2-yl)pentanoate de méthyle;
- le 3-hydroxy-3-(4-oxo-4,6-dihydroindolizino [1,2-*b*] quinoléin-2-yl)pentanoate de *tert*-butylcarbonyloxy-méthyle;
- la 2-[1-hydroxy-1-(1H-1,2,3,4-tétrazo-5-ylméthyl)propyl]-4,6-dihydroindolizino [1,2-*b*] quinoléin-4-one;
ou un sel pharmaceutiquement acceptable de ces derniers.

On préférera tout particulièrement pour l'invention l'acide 3-hydroxy-3-(4-oxo-4,6-dihydroindolizino [1,2-*b*] quinoléin-2-yl)pentanoïque ou un sel pharmaceutiquement acceptable de ce dernier.

L'invention a également pour objet un procédé de préparation des composés de formule générale (I) caractérisé en ce que l'on *N*-alkyle une pyridinone de formule générale A 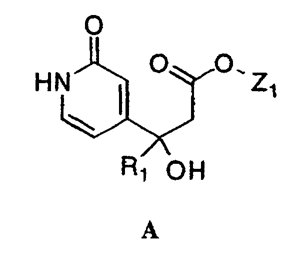 dans laquelle R₁ a la signification indiquée ci-dessus et le groupe Z₁ représente un radical alkyle inférieur, avec une quinoléine de formule générale B 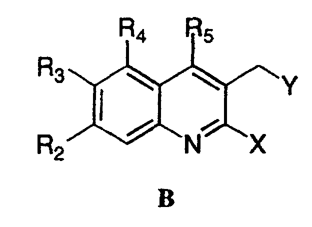 dans laquelle R₂, R₃, R₄ et R₅ ont la signification indiquée ci-dessus, X représente un atome de chlore, de brome ou d'iode et Y représente soit un atome de brome, soit un radical hydroxyle,
pour donner le composé de formule générale C 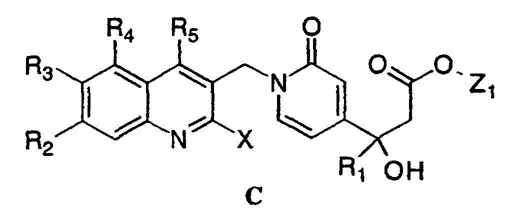 dans lequel R₁, R₂, R₃, R₄, R₅, X, et Z₁ ont la signification indiquée ci-dessus,
puis l'on cyclise le composé de formule générale C pour obtenir le composé de formule générale (I) dans laquelle R₁, R₂, R₃, R₄ et R₅ ont la signification indiquée ci-dessus, et R₁₀ représente un radical carbalkoxy.

Dans le procédé ci-dessus, la formation du composé C à partir des composés de formule générale A et B s'effectue, lorsque Y représente une fonction hydroxyle, par un traitement connu de l'homme de l'art sous le nom de réaction de Mitsunobu (se référer à Mitsunobu, O. et coll. *Synthesis,* p.1 (1981)). Il s'agit de déplacer la fonction hydroxyle du composé B par un nucléophile tel que le composé A, ou un dérivé déprotoné de ce dernier, par un traitement avec une phosphine, par exemple la triphénylphosphine, et un dérivé azodicarboxylate, par exemple l'azodicarboxylate de diéthyle, dans un solvant aprotique tel que, par exemple, le *N,N*-diméthylformamide ou le dioxane à une température de préférence comprise entre 0 °C et 60 °C telle que, par exemple, la température ambiante. La formation du composé C à partir des composés de formule générale A et B, lorsque Y représente un atome de brome, s'effectue par traitement du composé de formule générale B par un composé de formule générale A déprotoné dans un solvant aprotique tel que, par exemple, le tétrahydrofurane, le dioxane ou le *N,N*-diméthylformamide, à une température de préférence comprise entre 0 °C et 30 °C. La déprotonation du composé de formule générale A s'effectue par traitement avec un alkoxyde alcalin, un amidure alcalin ou un hydrure alcalin, tel que, par exemple, l'hydrure de sodium, dans un solvant aprotique tel que, par exemple le tétrahydrofurane, à une température de préférence comprise entre 0 °C et 30 °C. La cyclisation du composé C s'effectue de préférence en présence d'un catalyseur palladié (par exemple le diacétate de palladium) dans des conditions basiques (fournies par exemple par un acétate alcalin éventuellement combiné à un agent de transfert de phase tel que par exemple le bromure de tétrabutylammonium), dans un solvant tel que l'acétonitrile ou l'alcool amylique, à une température de préférence comprise entre 50° C et 120° C (R. Grigg et coll., *Tetrahedron* 46, page 4003 (1990)).

L'invention a également pour objet un procédé de préparation des composés de formule générale (I) caractérisé en ce que l'on *N*-alkyle une pyridinone de formule générale D 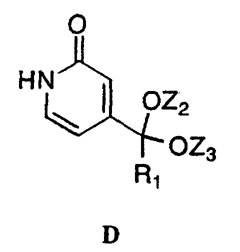 dans laquelle R₁ a la signification indiquée ci-dessus et les groupes Z₂ et Z₃ représentent, indépendamment, un radical alkyle inférieur ou Z₂ et Z₃ forment ensemble une chaîne hydrocarbonée saturée de 2 à 4 carbones, avec une quinoléine de formule générale B telle que définie ci-dessus pour donner le composé de formule générale E 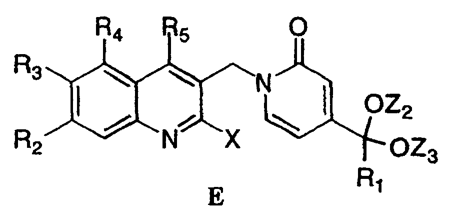 dans laquelle R₁, R₂, R₃, R₄, R₅, X, Z₂ et Z₃ ont la signification indiquée ci-dessus,
puis l'on cyclise le composé de formule générale E pour obtenir le composé de formule générale F 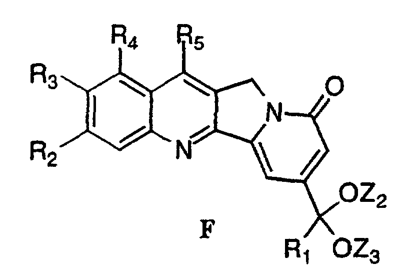 dans laquelle R₁, R₂, R₃, R₄, R₅, Z₂ et Z₃ ont la signification indiquée ci-dessus,
puis on libère la fonction carbonyle protégée du composés de formule générale F pour donner le composé de formule générale G 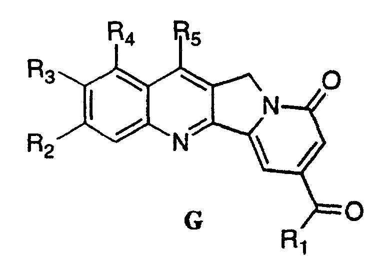 dans laquelle R₁, R₂, R₃, R₄, et R₅ ont la signification indiquée ci-dessus,
puis la fonction carbonyle du composé de formule générale G est traitée par un agent époxydant pour donner le composé de formule générale H 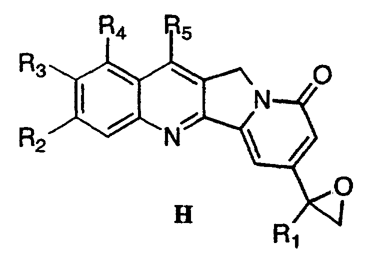 dans laquelle R₁, R₂, R₃, R₄, et R₅ ont la signification indiquée ci-dessus,
puis l'époxyde du composé de formule générale H est traité par un agent nitrilant pour donner le composé de formule générale (I), dans laquelle R₁, R₂, R₃, R₄, et R₅ ont la signification indiquée ci-dessus, et R₁₀ représente un radical cyano.

Dans le procédé ci-dessus, la formation du composé E à partir des composés de formule générale B et D s'effectue, lorsque Y représente une fonction hydroxyle, par un traitement connu de l'homme de l'art sous le nom de réaction de Mitsunobu (se référer à Mitsunobu, O. et coll. *Synthesis*, p.1 (1981)). Il s'agit de déplacer la fonction hydroxyle du composé B par un nucléophile tel que le composé D, ou un dérivé déprotoné de ce dernier, par un traitement avec une phosphine, par exemple la triphénylphosphine, et un dérivé azodicarboxylate, par exemple l'azodicarboxylate de diéthyle, dans un solvant aprotique tel que, par exemple, le tétrahydrofurane, à une température de préférence comprise entre 0 °C et 60 °C, par exemple à température ambiante. La formation du composé E à partir des composés de formule générale B et D, lorsque Y représente un atome de brome, s'effectue par traitement du composé de formule générale B par un composé de formule générale D déprotoné dans un solvant aprotique tel que, par exemple, le tétrahydrofurane, le dioxane ou le *N,N*-diméthylformamide, à une température de préférence comprise entre 0 °C et 30 °C. La déprotonation du composé de formule générale D s'effectue par traitement avec un alkoxyde alcalin, un amidure alcalin ou un hydrure alcalin, tel que, par exemple, l'hydrure de sodium, dans un solvant aprotique tel que, par exemple le tétrahydrofurane, à une température de préférence comprise entre 0 °C et 30 °C. La cyclisation du composé E s'effectue de préférence en présence d'un catalyseur palladié (par exemple le diacétate de palladium) dans des conditions basiques (fournies par exemple par un acétate alcalin éventuellement combiné à un agent de transfert de phase tel que par exemple le bromure de tétrabutylammonium), dans un solvant tel que l'acétonitrile ou l'alcool amylique, à une température comprise de préférence entre 50° C et 120° C (R. Grigg et coll., *Tetrahedron 46,* page 4003 (1990)). La libération de la fonction carbonyle protégée du composé de formule générale F pour donner le composé de formule générale G s'effectue par traitement dans des conditions acides telles que celles fournies, par exemple, par l'acide trifluoroacétique. L'époxydation donnant le composé de formule générale H s'effectue en traitant le composé de formule générale G par un ylure du soufre, obtenu par déprotonation d'un sel de trialkylsulfonium tel que, par exemple, l'iodure de triméthylsulfonium, par un alkoxyde alcalin tel que, par exemple, le *tert*-butylate de potassium, dans un solvant polaire aprotique tel que, par exemple le diméthylsulfoxyde, à une température de préférence comprise entre 0 °C et 30 °C. L'ouverture de l'époxyde du composé de formule générale H pour donner le composé de formule générale (I) dans laquelle R₁₀ représente un radical cyano est obtenue par traitement du composé de formule générale H avec un agent nitrilant tel que, par exemple, le cyanure de triméthylsilyle, en présence d'un acide de Lewis tel que, par exemple, le chlorure de diéthylaluminium, dans un solvant aprotique tel que, par exemple le dichlorométhane, à une température de préférence comprise entre 0 °C et 30 °C.

L'invention a également pour objet un procédé de préparation de composés de formule générale (I) caractérisé en ce que la fonction carbonyle d'un composé de formule générale G tel que définie ci-dessus est traitée par un agent alkylant approprié pour donner le composé de formule générale (I) correspondant. Ledit agent alkylant peut être fourni en utilisant des conditions de réaction connues de l'homme de l'art sous le nom de réaction de Réformatsky. Il s'agit de traiter un ester haloacétique tel que, par exemple, le bromoacétate de *tert*-butyle, ou un haloacétonitrile tel que, par exemple, le chloroacétonitrile, par un métal de transition tel que, par exemple, le zinc, dans un solvant aprotique tel que, par exemple, le tétrahydrofurane à une température de préférence comprise entre 0 °C et 60 °C. Ledit agent alkylant peut être également fourni par un énolate de lithium d'un ester acétique tel que, par exemple, l'acétate de *tert*-butyle traité par le diisopropylamidure de lithium dans un solvant aprotique tel que, par exemple, le tétrahydrofurane, à une température de préférence comprise entre -78 °C et la température ambiante.

L'invention a également pour objet un procédé de préparation de composés de formule générale (I) caractérisé en ce que la fonction ester d'un composé de formule générale (I) dans laquelle R₁₀ représente un radical carbalkoxy est hydrolysée pour donner un composé de formule générale (I) dans laquelle R₁₀ représente un radical carboxy. Cette transformation est généralement obtenue dans des condition alcalines fournies par une base alcaline telle que, par exemple, l'hydroxyde de lithium, dans un solvant polaire aqueux tel que, par exemple le méthanol aqueux ou encore un mélange tétrahydrofurane/méthanol/eau. Lorsque la fonction ester du composé de formule générale (I) à traiter est dérivée d'un alcool tertiaire tel que, par exemple, l'alcool *tert*-butylique, la saponification peut être obtenue dans des conditions acides fournies, par exemple par un acide minéral aqueux tel que, par exemple, l'acide chlorhydrique ou l'acide sulfurique, ou encore un acide organique fort tel que, par exemple, l'acide trifluoroacétique.

L'invention a également pour objet un procédé de préparation de composés de formule générale (I) caractérisé en ce que la fonction acide d'un composé de formule générale (I) dans laquelle R₁₀ représente un radical carboxy est estérifiée pour donner un composé de formule générale (I) dans laquelle R₁₀ représente un radical carbalkoxy. Une telle conversion est notamment obtenue par traitement de l'acide carboxylique de départ par un alcool approprié tel que, par exemple, l'éthanol, en présence d'un catalyseur acide tel que, par exemple, l'acide sulfurique concentré ou l'acide chlorhydrique concentré à une température de préférence comprise entre 40 °C et la température d'ébullition de l'alcool considéré. On peut également traiter l'acide carboxylique activé par un agent tel que, par exemple le carbonyldiimidazole ou le chlorure de thionyle ou le dicyclohexylcarbodiimide, avec un alcool. Enfin, est aussi inclus dans ce procédé d'estérification le traitement du groupe carboxylique par une base telle que, par exemple, la soude ou le carbonate de potassium, dans un solvant polaire tel que, par exemple, le diméthylsulfoxyde ou le *N,N*-diméthylformamide, suivi d'un électrophile alkylant tel que, par exemple, l'iodure de méthyle ou le pivaloate de chloraméthyle.

L'invention a encore pour objet un procédé de préparation de composés de formule générale (I) caractérisé en ce que la fonction ester d'un composé de formule générale (I) dans laquelle R₁₀ représente un radical carbalkoxy est transestérifiée pour donner un composé de formule générale (I) dans laquelle R₁₀ représente un autre radical carbalkoxy. La transestérification peut être obtenue par un traitement dans l'alcool dont est dérivé l'ester désiré, en présence d'une catalyse acide fournie, par exemple, par l'acide sulfurique concentré ou l'isopropoxyde de titane, à une température de préférence comprise entre 40 °C et la température de reflux de l'alcool considéré.

L'invention a également pour objet un procédé de préparation de composés de formule générale (I) caractérisé en ce que la fonction nitrile d'un composé de formule générale (I) dans laquelle R₁₀ représente un radical cyano subit une addition dipolaire avec un azoture pour donner un composé de formule générale (I) dans laquelle R₁₀ représente un radical 1,2,3,4-tétrazole-5-yle. Cette dernière transformation est obtenue par traitement du composé cyano par un azoture tel que, par exemple l'azoture de triméthylsilyle, en présence d'un catalyseur tel que, par exemple, l'oxyde de dibutylétain, dans un solvant aprotique tel que, par exemple, le toluène, à une température de préférence comprise entre 50 °C et 110 °C, par exemple la température de reflux du toluène.

Dans tous les procédés de l'invention décrits ci-dessus, les fonctions portées par les groupes R₂, R₃, R₄ et R₅ peuvent être protégées et déprotégées si nécessaire selon les méthodes classiques de protection-déprotection connues de l'homme de l'art (Greene, T., Protective Groups in Organic Synthesis 10-86 (John Wiley & Sons 1981)). Une illustration de ce concept de protection-déprotection est donnée par l'exemple 1 ci-dessous : le produit de départ est une aniline de formule générale L où R₃ est un radical méthoxy et le produit final répond à un composé de formule générale (I) où R₃ est un radical benzyloxy.

Les pyridinones de formule générale A sont nouvelles. Elles peuvent être préparées selon un procédé caractérisé en ce que l'on déprotège une 2-alkoxypyridine de formule générale J 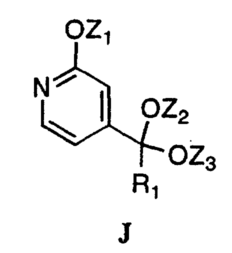 dans laquelle R₁, Z₁, Z₂ et Z₃ ont la signification indiquée ci-dessus, pour donner la pyridinone de formule générale K 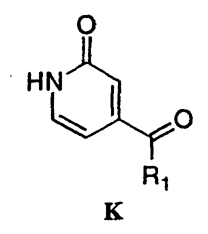 dans laquelle R₁ a la signification indiquée ci-dessus;
puis l'on traite par un agent alkylant fonctionnalisé le composé de formule générale K pour obtenir un composé de formule générale A dans laquelle R₁ et Z₁ ont la signification indiquée ci-dessus.

Les 2-alkoxypyridines de formule générale J peuvent être obtenues, par exemple, selon un procédé décrit dans la demande PCT/FR96/00980. La déprotection des composés de formule générale J peut être obtenue soit par un traitement par un acide minéral dilué tel que, par exemple, l'acide chlorhydrique normal, à une température de préférence comprise entre 60 °C et 120 °C telle que, par exemple, la température de reflux. On peut également effectuer la déprotection des composés de formule générale J par un traitement avec un agent désalkylant tel que, par exemple, le tribromure de bore ou l'iodure de triméthylsilyle (éventuellement généré *in situ*), dans un solvant aprotique tel que, par exemple, le dichlorométhane ou l'acétonitrile, à une température de préférence choisie entre 40 °C et 90 °C ou, par exemple, la température de reflux du solvant. Le traitement du composé de formule générale K par un agent alkylant fonctionnalisé peut être effectué dans des conditions de réactions connues de l'homme de l'art sous le nom de réaction de Réformatsky. Il s'agit de traiter un ester haloacétique tel que, par exemple, le bromoacétate de *tert*-butyle, ou un haloacétonitrile tel que, par exemple, le chloroacétonitrile, par un métal de transition tel que, par exemple, le zinc, dans un solvant aprotique tel que, par exemple, le tétrahydrofurane à une température de préférence comprise entre 0 °C et 60 °C. Un agent alkylant approprié peut également être fourni par un énolate de lithium d'un ester acétique tel que, par exemple, l'acétate de *tert*-butyle traité par le diisopropylamidure de lithium dans un solvant aprotique tel que, par exemple, le tétrahydrofurane, à une température de préférence comprise entre -78 °C et la température ambiante.

Les pyridinones de formule générale D sont nouvelles. Elles peuvent être obtenues selon un procédé caractérisé en ce que l'on protège la fonction cétonique dans le composé de formule générale K pour obtenir un composé de formule générale D dans laquelle R₁, Z₂ et Z₃ ont la signification indiquée ci-dessus. Une telle protection obtenue par des conditions de réaction classiques et connues de l'homme de l'art sous le nom d'acétalisation (Greene, T., Protective Groups in Organic Synthesis 10-86 (John Wiley & Sons 1981)).

Les quinoléines de formule générale B peuvent être obtenues à partir d'anilines de formule générale L 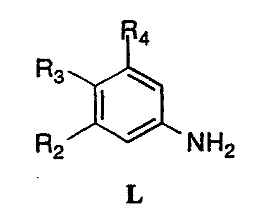 dans laquelle R₂, R₃ et R₄ ont la signification indiquée dans les formules générales des composés (I), ou encore sont des précurseurs de ces derniers dans le cadre de l'emploi des techniques de protection-déprotection. Ainsi, dans les procédés ci-dessous, les groupes R₂, R₃ et R₄ peuvent être protégés si nécessaire selon les méthodes classiques de protection (Greene, T., Protective Groups in Organic Synthesis 10-86 (John Wiley & Sons 1981)). Une illustration de ce concept de protection-déprotection est donnée par l'exemple 1 ci-dessous: le produit de départ est une aniline de formule L où R₃ est un radical méthoxy et le produit final répond à un composé formule (I) où R₃ est un radical benzyloxy.

Les quinoléines de formule B peuvent être obtenues selon le procédé suivant : les anilines de formule générale L telles que définies ci-dessus sont *N*-acétylées par traitement avec un agent acétylant tel que, par exemple, l'anhydride acétique. Les acétanilides ainsi obtenus sont traités à une température comprise de préférence entre 50 °C et 100 °C, plus préférentiellement à 75 °C, par un réactif connu de l'homme de l'art sous de nom de réactif de Vilsmeyer (obtenu par l'action de l'oxychlorure de phosphoryle sur la *N,N*-diméthylformamide à une température comprise de préférence entre 0 °C et 10 °C) pour donner le 2-chloro-3-quinoléinecarbaldéhyde correspondant (se référer, par exemple à Meth-Cohn, et coll. *J. Chem. Soc., Perkin Trans. I* p.1520 (1981); Meth-Cohn, et coll. *J. Chem. Soc., Perkin Trans. I* p.2509 (1981); et Nakasimhan et coll. *J. Am. Chem. Soc., 112,* p.4431 (1990)). Le chlore en position 2 des 2-chloro-3-quinoléinecarbaldéhydes peut être substitué par de l'iode ou du brome en chauffant le produit dans un solvant inerte tel que, par exemple, l'acétonitrile en présence d'un sel d'iode ou de brome (par exemple l'iodure de sodium ou le bromure de tétrabutylammonium). Une trace d'acide tel que l'acide chlorhydrique concentré peut être nécessaire pour catalyser cette transformation. Les 2-halo-3-quinoléinecarbaldéhydes sont facilement réduits en 2-halo-3-quinoléineméthanols correspondants de formule générale B où Y représente une fonction hydroxyle, dans des conditions classiques connues de l'homme de l'art telles que le traitement dans un solvant alcoolique (par exemple le méthanol) par du borohydrure de sodium à une température de préférence comprise entre 0° C et 40° C. Les 2-halo-3-quinoléineméthanols de formule générale B où Y représente une fonction hydroxyle peuvent être convertis en 3-bromométhyl-2-haloquinoléines de formule générale B où Y représente un atome de brome par un traitement avec du tétrabromométhane en présence d'une phosphine telle que, par exemple, la triphénylphosphine, dans un solvant aprotique chloré tel que, par exemple, le dichlorométhane ou le 1,2-dichloroéthane à température ambiante.

Les quinoléines de formule B peuvent également être obtenues selon le procédé suivant : les anilines de formule générale L telles que définies ci-dessus sont acylées par réaction avec un nitrile (tel que le chloroacétonitrile ou le propionitrile) en présence de trichlorure de bore et d'un autre acide de Lewis tel que le trichlorure d'aluminium, le tétrachlorure de titane ou le chlorure de diéthylaluminium dans un solvant aprotique ou un mélange de solvant aprotique, suivie d'une hydrolyse (*cf*. Sugasawa, T, et coll. *J. Am. Chem. Soc. 100,* p. 4842 (1978)). L'intermédiaire ainsi obtenu est ensuite traité par le chlorure d'éthylmalonyle dans un solvant aprotique tel que l'acétonitrile en présence d'une base telle que la triéthylamine, puis traité par un alcoolate alcalin, par exemple l'éthylate de sodium dans l'éthanol, pour donner un 2-hydroxy-3-quinoléinecarboxylate d'éthyle substitué en position 4. Ce dernier est transformé en 2-chloro-3-quinoléinecarboxylate d'éthyle par un traitement avec de l'oxychlorure de phosphoryle. Lorsque la position 4 de la quinoléine porte un groupe chlorométhyle, on peut effectuer une substitution nucléophile par traitement avec une amine secondaire telle que par exemple la diméthylamine, la N-méthylpipérazine, la morpholine ou la pipéridine. Le 2-chloro-3-quinoléinecarboxylate d'éthyle est ensuite réduit par l'hydrure de diisobutylaluminium dans un solvant aprotique tel que le dichlorométhane pour donner le 2-chloro-3-quinoléineméthanol de formule générale B. Des analogues des quinoléines intermédiaires B ont été décrits dans la littérature et en particulier dans la demande PCT 95/05427.

L'invention a également pour objet, à titre de produits industriels nouveaux, et notamment à titre de produits industriels nouveaux destinés à la préparation des produits de formule générale (I), les produits de formules A, D et K telles que décrites ci-dessus.

Certains composés de l'invention peuvent être préparés sous la forme de sels pharmaceutiquement acceptables selon les méthodes usuelles. Des sels acceptables comprennent, à titre d'exemple et de façon non limitative, des sels d'addition d'acides inorganiques tels que chlorhydrate, sulfate, phosphate, diphosphate, bromhydrate et nitrate ou d'acides organiques tels que acétate, maléate, fumarate, tartrate, succinate, citrate, lactate, méthane sulfonate, p-toluènesulfonate, pamoate, salicylate, oxalate et stéarate. Entrent également dans le champ d'application de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical Salts", F.M. Berge, J. Pharm. Sci. 66:1 (1977).

Les composés de la présente invention possèdent d'intéressantes propriétés pharmacologiques. C'est ainsi que les composés de la présente invention présentent une activité inhibitrice de la topoisomérase I et/ou une activité inhibitrice de la topoisomérase II. L'état de la technique suggère que les composés de l'invention possèdent une activité anti-tumorale, une activité anti-parasitaire et une activité anti-virale. Les composés de la présente invention peuvent ainsi être utilisés dans différentes applications thérapeutiques.

On trouvera ci-après, dans la partie expérimentale, une illustration des propriétés pharmacologiques des composés de l'invention.

Les composés peuvent inhiber les topoisomérases de type I et/ou de type II, chez un patient, par exemple un mammifère tel que l'homme, par administration à ce patient d'une quantité thérapeutiquement efficace d'un composé de formule générale (I).

Les composés de l'invention possèdent également une activité anti-tumorale. Ils peuvent être utilisés pour le traitement des tumeurs, par exemple des tumeurs exprimant une topoisomérase, chez un patient par administration à ce dernier d'une quantité thérapeutiquement efficace d'un composé de formule générale (I). Des exemples de tumeurs ou de cancers comprennent les cancers de l'oesophage, de l'estomac, des intestins, du rectum, de la cavité orale, du pharynx, du larynx, du poumon, du colon, du sein, du *cervix uteri*, du *corpus endometrium*, des ovaires, de la prostate, des testicules, de la vessie, des reins, du foie, du pancréas, des os, des tissus conjonctifs, de la peau, des yeux, du cerveau et du système nerveux central, ainsi que le cancer de la thyroïde, la leucémie, la maladie de Hodgkin, les lymphomes autres que ceux de Hodgkin, les myélomes multiples et autres.

Ils peuvent également être utilisés pour le traitement des infections parasitaires par l'inhibition des hémoflagellates (par exemple dans la trypanosomie ou les infections de type leishmania) ou par inhibition de la plasmodie (comme par exemple dans la malaria), mais aussi le traitement des infections ou maladies virales.

Ces propriétés rendent les produits de formule générale (I) aptes à une utilisation pharmaceutique. La présente demande a donc également pour objet, à titre de médicaments, les composés de formule générale (I) telles que définies ci-dessus, ainsi que les sels d'addition avec les acides minéraux ou organiques pharmaceutiquement acceptables de produits de formule générale (I), ainsi que les compositions pharmaceutiques contenant, à titre de principe actif, l'un au moins des composés tels que définis ci-dessus.

L'invention concerne ainsi des compositions pharmaceutiques contenant un composé de formule générale (I) telle que définie précédemment ou un sel pharmaceutiquement acceptable de celui-ci, en association avec un support pharmaceutiquement acceptable choisi en fonction du mode d'administration (par exemple administration orale, intraveineuse, intrapéritonéale, intramusculaire, trans-dermique ou sous-cutanée). La composition pharmaceutique (par exemple thérapeutique) peut être sous forme solide, liquide, de liposomes ou de micelles lipidiques.

La composition pharmaceutique peut être sous forme solide comme, par exemple, les poudres, pilules, granules, comprimés, liposomes, gélules ou suppositoires. La pilule, le comprimé ou la gélule peuvent être revêtus d'une substance capable de protéger la composition de l'action de l'acide gastrique ou des enzymes dans l'estomac du sujet pendant une période de temps suffisante pour permettre à cette composition de passer non digérée dans l'intestin grêle de ce dernier. Le composé peut aussi être administré localement, par exemple à l'emplacement même de la tumeur. Le composé peut aussi être administré selon le processus de la libération prolongée (par exemple une composition à libération prolongée ou une pompe d'infusion). Les supports solides appropriés peuvent être, par exemple, le phosphate de calcium, le stéarate de magnésium, le carbonate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire. Les compositions pharmaceutiques contenant un composé de l'invention peuvent donc également se présenter sous forme liquide comme, par exemple, des solutions, des émulsions, des suspensions ou une formulation à libération prolongée. Les supports liquides appropriés peuvent être, par exemple, l'eau, les solvants organiques tels que le glycérol ou les glycols tel que le polyéthylène glycol, de même que leurs mélanges, dans des proportions variées, dans l'eau.

L'invention a également pour objet l'utilisation des produits de formule (I) telle que définie ci-dessus, pour la préparation de médicaments destinés à inhiber les topoisomérases, et plus particulièrement à inhiber les topoisomérases de type I, ou les topoisomérases de type II, ou, simultanément, les deux types de topoisomérases, pour la préparation de médicaments destinés à traiter les tumeurs, pour la préparation de médicaments destinés à traiter les infections parasitaires, ainsi que pour la préparation de médicaments destinés à traiter des infections ou maladies virales.

La dose d'un composé selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnés ici sont incorporés par référence.

### PARTIE EXPÉRIMENTALE :

### Exemple 1 : 3-(9-benzyloxy-10-fluoro-4-oxo-4,6-dihydroindolizino [1,2-b]quinoléin-2-yl)-3-hydroxypentanoate de tert-butyle

### 1.a. N-(3-fluoro-4-méthoxyphényl)acétamide :

Un mélange de 3-fluoro-4-méthoxyaniline (56,4 g; 400 mmol) et de triéthylamine (56 ml; 400 mmol) dans du dichlorométhane (400 ml) est refroidi au moyen d'un bain de glace. De l'anhydride acétique (57 ml; 600 mmol) est ajouté goutte à goutte et le mélange réactionnel est agité pendant 3 h à température ambiante. Le milieu réactionnel est ensuite lavé successivement avec de l'eau, avec une solution aqueuse de bicarbonate de sodium à 10 % puis avec une solution aqueuse saturée en chlorure de sodium. La fraction organique est séchée sur du sulfate de sodium et concentrée sous pression réduite. Le résidu est recristallisé dans un mélange acétate d'éthyle/pentane pour donner 66,5 g (91 %) d'un solide blanc, p.f. 120 °C.

RMN ¹H (CDCl₃) : 2,15 (s, 3H); 3,86 (s, 3H); 6,92 (dd, 1H); 7,13 (dd, 1H); 7,40 (dd, 1H); 7,55 (br, 1H).

### 1.b. 2-chloro-7-fluoro-6-méthoxy-3-quinoléinecarbaldéhyde :

Du *N*-(3-fluoro-4-méthoxyphényl)acétamide (obtenu selon **1.a,** 30 g, 164 mmol) est ajouté au réactif de Vilsmeyer (obtenu, sous atmosphère d'argon, par addition goutte à goutte d'oxychlorure de phosphoryle (75 ml, 800 mmol) à du *N,N*-diméthylformamide anhydre (25 ml, 320 mmol) refroidi avec un bain de glace, puis agité pendant 0,5 h) et le mélange résultant est chauffé à 75 °C pendant 2 h. Après refroidissement à température ambiante, le milieu réactionnel est ajouté goutte à goutte à un mélange de glace et d'eau (500 ml). La suspension jaune ainsi obtenue est maintenue sous agitation durant 1 h. Le précipité obtenu est ensuite filtré, lavé à l'eau jusqu'à pH neutre puis séché sous pression réduite en présence de pentoxyde de phosphore pour donner 13,6 g (35 %) d'un solide beige, p.f. 180 °C.

RMN ¹H (DMSO): 4,05 (s, 3H); 7,30 (d, 1H); 7,74 (d, 1H); 8,16 (s, 1H); 10,52 (s, 1H).

### 1.c. 2-chloro-7-fluoro-6-hydoxy-3-quinoléinecarbaldéhyde :

Du 2-chloro-7-fluoro-6-méthoxy-3-quinoléinecarbaldéhyde (obtenu selon **1.b,** 27,2 g, 113 mmol) suspendu dans du dichlorométhane anhydre (980 ml) est traité goutte à goutte par du tribromure de bore (solution molaire dans le dichlorométhane, 340 ml, 340 mmol) et le mélange résultant est agité à température ambiante pendant 24 h puis refroidi à 0 °C. Le milieu réactionnel est ajouté au goutte à goutte à un mélange eau/glace (500 ml) en agitation, le solide formé est filtré puis séché sous pression réduite pour donner un solide jaune (13,5 g, 53 %), p.f. 270 °C.

RMN ¹H (DMSO): 7,64 (d, 1H); 7,84 (d, 1H); 8,84 (s, 1H); 10,33 (s, 1H); 11,15 (s, 1H).

### 1.d. 6-benzyloxy-2-chloro-7-fluoro-3-quinoléinecarbaldéhyde :

A une solution de 2-chloro-7-fluoro-6-hydoxy-3-quinoléinecarbaldéhyde (obtenu selon **1.c,** 13 g, 58 mmol) dans du *N,N*-diméthylformamide anhydre (120 ml) est ajouté du carbonate de potassium (9,2g, 66 mmol) et le mélange, placé sous atmosphère d'argon, est refroidi à 0 °C. Du bromure de benzyle (7,9 ml, 66 mmol) est ajouté goutte à goutte et le milieu réactionnel est maintenu sous agitation à température ambiante pendant 16 h. Le milieu réactionnel est alors coulé sur de l'eau glacée (200 ml) et le solide jaune résultant est filtré, puis repris dans de l'éthanol (200 ml) et concentré sous pression réduite. Le résidu est repris dans de l'éther éthylique (200 ml) et le solide jaune résultant est filtré puis séché sous pression réduite pour donner 15 g (82 %) d'un solide jaune, p.f. 228 °C.

RMN ¹H (CDCl₃) : 5,29 (s, 2H); 7,2-7,6 (m, 6H); 7,72 (d, 1H); 8,60 (s, 1H); 10,52 (s, 1H).

### 1.e. 6-benzyloxy-7-fluoro-2-iodo-3-quinoléinecarbaldéhyde :

Une suspension de 6-benzyloxy-2-chloro-7-fluoro-3-quinoléinecarbaldéhyde (obtenu selon **1.d,** 15 g, 47 mmol) et d'iodure de sodium (18 g, 120 mmol) dans de l'acétonitrile anhydre (500 ml) est traitée par une quantité catalytique d'acide chlorhydrique concentré (1,2 ml), puis portée au reflux sous argon pendant 8 h. Le mélange réactionnel est alors concentré à 20 % du volume initial, puis traité avec une solution aqueuse à 10 % de bicarbonate de sodium jusqu'à pH neutre, puis filtré et lavé successivement à l'eau, à l'éthanol et à l'éther éthylique pour donner 13 g (68 %) d'un solide jaune, p.f. 210 °C.

RMN ¹H (CDCl₃) : 5,28 (s, 2H); 7,43 (d, 1H); 7,43 (m, 5H); 7,74 (d, 1H); 8,39 (s, 1H); 10,21 (s, 1H).

### 1.f. 6-benzyloxy-7-fluoro-2-iodo-3-quinolylméthanol :

Une suspension de 6-benzyloxy-7-fluoro-2-iodo-3-quinoléinecarbaldéhyde (obtenu selon **1.e,** 13 g, 32 mmol) dans du méthanol (100 ml) est traitée par du borohydrure de sodium (1,85 g, 48 mmol). Après 1 h de réaction, le milieu réactionnel est concentré sous pression réduite puis repris à l'eau, filtré, lavé à l'eau et à l'éthanol pour donner, après séchage sous pression réduite, 10 g (76 %) d'un solide blanc, p.f. 188 °C.

RMN ¹H (CDCl₃) : 4,61 (d, 2H); 5,19 (s, 2H); 5,32 (t, 1H); 7,21 (d, 1H); 7,62 (d, 1H); 8,09 (s, 1H).

### 1.g . 1-(2-oxo-1,2-dihydro-4-pyridinyl)-1-propanone :

Un mélange de 4-(2-éthyl-1,3-dioxan-2-yl)-2-méthoxypyridine (préparée selon la méthode décrite dans la demande de brevet PCT/FR96/00980, 57 g, 255 mmol) et d'iodure de sodium (88 g, 580 mmol) dans de l'acétonitrile (1l) est traité par du chlorure de triméthylsilane (74 ml, 586 mmol), porté au reflux 3 h, puis agité à température ambiante pendant 16 h. Le milieu réactionnel est alors traité avec de l'eau (100 ml) et concentré à sec après élimination des insolubles par filtration. Le résidu est repris par de l'acétate d'éthyle et lavé successivement à l'eau et avec une solution aqueuse saturée en chlorure de sodium. La phase organique est séchée, concentrée sous pression réduite, et le résidu est repris dans l'éther diéthylique pour donner après filtration et séchage 30 g (88 %) d'un solide blanc, p.f. 168 °C.

RMN ¹H (CDCl₃) : 1,22 (t, 3H); 2,96 (q, 2H); 6,88 (d, 1H); 7,05 (s, 1H); 7,49 (d, 1H); 13,2 (br, 1H).

### 1.h. 3-hydroxy-3-(2-oxo-1,2-dihydro-4-pyridinyl)pentanoate de tert-butyle :

Une solution de diisopropylamine (35 ml, 250 mmol) dans du tétrahydrofurane anhydre (275 ml) est traitée goutte à goutte à 0 °C, sous argon, par du n-butyllithium (2,5 M dans l'hexane, 100 ml, 250 mmol). Le mélange résultant est agité à 0°C pendant 15 min, puis refroidi à -78 °C et traité par de l'acétate de *tert-*butyle (33,8 ml, 250 mmol). Après agitation à -78 °C pendant 15 min, le réactif lithié résultant est ajouté goutte à goutte, durant 1 h, au moyen d'une canule de transfert, sur une solution à -78 °C de 1-(2-oxo-1,2-dihydro-4-pyridinyl)-1-propanone (obtenue selon **1.g,** 15,2 g, 100 mmol) dans du tétrahydrofurane anhydre (330 ml) et le mélange résultant est maintenu à -78 °C pendant 15 min, puis laissé remonter à 0 °C durant 1 h. Le milieu réactionnel est hydrolysé par addition d'eau (60 ml) puis les volatiles sous évaporés sous pression réduite. Le résidu est repris à l'acétate d'éthyle et la solution résultante est lavée à l'eau, séchée et concentrée. Le résidu est suspendu dans l'éther diéthylique et filtré pour donner après séchage 21,5 g (80 %) d'un solide blanc, p.f. 167 °C.

RMN ¹H (DMSO): 0,67 (t, 3H); 1,25 (s, 9H); 1,70 (q, 2H); 2,59 (dd, 2H); 4,97 (s, 1H); 6,18 (d, 1H); 6,32 (s, 1H); 7,23 (d, 1H); 11,3 (br, 1H).

### 1.i. 3-[1-(6-benzyloxy-7-fluoro-2-iodo-3-quinolylméthyl)-2-oxo-1,2-dihydro-4-pyridinyl]-3-hydroxypentanoate de tert-butyle :

Un mélange, sous atmosphère d'argon, de 6-benzyloxy-7-fluoro-2-iodo-3-quinolylméthanol (obtenu selon **1.f,** 2,05 g, 5 mmol), de 3-hydroxy-3-(2-oxo-1,2-dihydro-4-pyridinyl)pentanoate de *tert*-butyle (obtenu selon **1.h,** 1,47 g, 5,5 mmol), et de tributylphosphine (1,36 ml, 5,5 mmol) dans du tétrahydrofurane anhydre (20 ml) est traité goutte à goutte par de l'azodicarboxylate de diéthyle (1,3 ml, 7,5 mmol). Le mélange réactionnel est ensuite agité à température ambiante pendant 6 h, puis concentré sous pression réduite. Le résidu huileux résultant est repris dans du dichlorométhane (100 ml) et lavé par du chlorure d'ammonium aqueux saturé puis du chlorure de sodium aqueux saturé. La phase organique est séchée sur sulfate de sodium puis concentrée à 5 ml et additionnée d'acétonitrile pour obtenir un précipité blanc qui est gardé à 4°C durant 16 h. Le précipité est recueilli par filtration puis lavé à l'éther isopropylique pour donner 1,8 g (55 %) d'un solide blanc, p.f. 174 °C.

RMN ¹H (DMSO): 0,73 (t, 3H); 1,27 (s, 9H); 1,78 (m, 2H); 2,67 (dd, 2H); 5,12 (s, 2H); 5,26 (s, 2H); 6,40 (d, 1H); 6,52 (s, 1H); 7,3-7,6 (m, 7H); 7,72 (m, 1H); 7,86 (m, 1H).

### 1.j. 3-(9-benzyloxy-10-fluoro-4-oxo-4,6-dihydroindolizino [1,2-b] quinoléin-2-yl)-3-hydroxypentanoate de tert-butyle :

Un mélange de 3-[1-(6-benzyloxy-7-fluoro-2-iodo-3-quinolylméthyl)-2-oxo-1,2-dihydro-4-pyridinyl]-3-hydroxypentanoate de *tert*-butyle (obtenu selon **1.i,** 1,6 g, 2,4 mmol), de bromure de tétrabutylammonium (0,77 g, 2,4 mmol), d'acétate de potassium (0,24 ml, 2,4 mmol) et d'acétate de palladium (0,55 g, 2,4 mmol) dans de l'alcool amylique anhydre (30 ml) est chauffé à 80 °C sous atmosphère d'argon pendant 3 h, puis concentré sous pression réduite. Le résidu est repris dans du méthanol (50 ml) et du dichlorométhane (100 ml), filtré sur célite puis concentré sous pression réduite pour donner un solide rosé qui est repris au méthanol, puis traité à chaud au charbon actif. La liqueur obtenue par filtration est concentrée à 5 ml, puis placée à 4 °C durant 16 h. Le précipité résultant est recueilli par filtration et lavé à l'éther diéthylique pour donner, après séchage, 370 mg (29 %) d'un solide blanc, p.f. > 275 °C.

RMN ¹H (DMSO): 0,73 (t, 3H); 1,22 (s, 9H); 1,83 (m, 2H); 2,77 (dd, 2H); 5,21 (s, 2H); 5,25 (s, 1H); 5,37 (s, 2H); 6,59 (s, 1H); 7,21 (s, 1H); 7,4-7,5 (m, 3H); 7,57 (d, 2H); 7,88 (d, 1H); 7,95 (d, 1H); 8,56 (s, 1H).

### Exemple 2 : 3-(10-fluoro-9-méthoxy-4-oxo-4,6-dihydroindolizino [1,2-b] quinoléin-2-yl)-3-hydroxypentanoate de tert-butyle :

On applique les procédures **1.e, 1.f, 1.i** et **1.j** en partant du 2-chloro-7-fluoro-6-méthoxy-3-quinoléinecarbaldéhyde (obtenu selon **1.b**) au lieu du 6-benzyloxy-2-chloro-7-fluoro-3-quinoléine-carbaldéhyde. On obtient un solide blanc; p.f. 247 °C.

RMN ¹H (DMSO): 0,72 (t, 3H); 1,22 (s, 9H); 1,85 (m, 2H); 2,77 (dd, 2H); 4,02 (s, 3H); 5,20 (s, 2H); 5,27 (s, 1H); 6,59 (s, 1H); 7,20 (s, 1H); 7,73 (d, 1H); 7,91 (d, 1H); 8,55 (s, 1H).

### Exemple 3 : 3-hydroxy-3-(7-méthyl -4-oxo-4,6-dihydroindolizino [1,2-b] quinoléin-2-yl)pentanoate de tert-butyle :

### 3.a. 4-méthyl-2-oxo-1,2-dihydro-3-quinoléinecarboxylate d'éthyle :

A une solution de 2-aminoacétophénone (10,5 g, 78 mmol) et de triéthylamine (13,9 ml, 100 mmol) dans de l'acétonitrile anhydre (110 ml), sous argon et à 0 °C, est ajoutée goutte à goutte une solution de chlorure d'éthylmalonyle (12,9 ml, 100 mmol) dans de l'acétonitrile anhydre (30 ml). Le milieu réactionnel est réchauffé à température ambiante puis traité au goutte à goutte et sous argon par une solution d'éthylate de sodium (obtenue par 1,8 g, 78 mmol, de sodium dans 80 ml d'éthanol), puis laissé sous agitation 12 h à température ambiante. Le mélange réactionnel est alors versé dans de l'eau glacée (100 ml) et agité deux heures, puis filtré. Le précipité ainsi recueilli est lavé à l'eau, à l'éthanol et à l'éther pour donner 15,2 g (84 %) d'un solide blanc.

RMN ¹H (DMSO) : 1,30 (t, 3H); 2,40 (s, 3H); 4,31 (q, 2H); 7,24 (t, 1H); 7,37 (d, 1H); 7,4 (br, 1H); 7,58 (t, 1H); 7,81 (d, 1H).

### 3.b. 2-chloro-4-méthyl-3-quinoléinecarboxylate d'éthyle :

Une suspension de 4-méthyl-2-oxo-1,2-dihydro-3-quinoléinecarboxylate d'éthyle (obtenu selon **3.a**, 15,2 g, 0,066 mol) dans du chlorure de phosphoryle (243 ml) est portée au reflux pendant 6 h. Le chlorure de phosphoryle est évaporé sous pression réduite sans aller à sec, et le résidu visqueux est coulé sur de l'eau glacée (300 ml). Le précipité ainsi obtenu est filtré, lavé à l'eau jusqu'à pH neutre, puis lavé à l'éthanol et à l'éther diéthylique pour donner après séchage 8,8 g (53 %) d'un solide blanc, p.f. 110°C.

RMN ¹H (CDCl₃) : 1,45 (t, 3H); 2,67 (s, 3H); 4,51 (q, 2H); 7,61 (t, 1H); 7,76 (t, 1H); 8,00 (m, 2H).

### 3.c. 2-chloro-4-méthyl-3-quinoléineméthanol :

Une solution sous argon de 2-chloro-4-méthyl-3-quinoléinecarboxylate d'éthyle (obtenu selon **3.b,** 8,75 g, 35 mmol) dans du dichlorométhane anhydre (200 ml) est traitée goutte à goutte, à température ambiante, par de l'hydrure de diisobutylaluminium (1M dans du dichlorométhane, 65 ml, 65 mmol), puis chauffée à 40 °C pendant 4 h. Le milieu réactionnel est alors refroidi à 0 °C, puis traité avec précaution par une solution aqueuse de sel de Rochelle à 20 % (105 ml) et du dichlorométhane (200 ml) et maintenu sous agitation pendant 1 h. La phase organique est alors décantée, lavée trois fois à l'eau, puis séchée sur sulfate de magnésium et concentrée sous pression réduite. Le résidu est purifié par chromatographie sur colonne (SiO₂, acétate d'éthyle/heptane: 5/95 à 50/50) pour donner 6 g (82 %) d'un solide blanc.

RMN ¹H (CDCl₃): 2,24 (br, 1H); 2,81 (s, 3H); 5,04 (d, 2H); 7,58 (t, 1H); 7,71 (t, 1H); 7,99 (m, 2H).

### 3. d. 3-[1-(2-chloro-4-méthyl-3-quinolylméthyl)-2-oxo-1,2-dihydro-4-pyridinyl]-3-hydroxypentanoate de tert-butyle :

Un mélange, sous atmosphère d'argon, de 2-chloro-4-méthyl-3-quinoléineméthanol (obtenu selon **3.c,** 2,08 g, 10 mmol), de 3-hydroxy-3-(2-oxo-1,2-dihydro-4-pyridinyl)pentanoate de *tert*◇butyle (obtenu selon **1.h,** 2 g, 11 mmol), et de tributylphosphine (2,75 ml, 11 mmol) dans du tétrahydrofurane anhydre (40 ml) est traité goutte à goutte par de l'azodicarboxylate de diéthyle (2,6 ml, 15 mmol). Le mélange réactionnel est ensuite agité à température ambiante pendant 6 h, puis concentré sous pression réduite. Le résidu huileux résultant est repris dans du dichlorométhane (200 ml) et lavé par du chlorure d'ammonium aqueux saturé puis du chlorure de sodium aqueux saturé. La phase organique est séchée sur sulfate de sodium puis concentrée à 5 ml et additionnée d'acétonitrile pour obtenir un précipité blanc qui est gardé à 4°C durant 16 h. Le précipité est recueilli par filtration puis lavé à l'éther isopropylique pour donner 1,8 g (39 %) d'un solide blanc.

RMN ¹H (DMSO) : 0,73 (t, 3H); 1,28 (s, 9H); 1,75 (q, 2H); 2,66 (dd, 2H); 2,80 (s, 3H); 5,11 (s, 1H); 5,44 (dd, 2H); 6,29 (d, 1H); 6,49 (s, 1H); 6,70 (s, 1H); 7,29 (d, 1H); 7,80 (t, 1H); 7,96 (t, 1H); 8,05 (d, 1H); 8,32 (d, 1H).

### 3.e. 3-hydroxy-3-(7-méthyl-4-oxo-4,6-dihydroindolizino [1,2-b] quinoléin-2-yl)pentanoate de tert-butyle :

Un mélange de 3-[1-(2-chloro-4-méthyl-3-quinolylméthyl)-2-oxo-1,2-dihydro-4-pyridinyl]-3-hydroxypentanoate de *tert*-butyle (obtenu selon **3.d,** 1,29 g, 2,8 mmol), de bromure de tétrabutylammonium (0,99 g, 3,1 mmol), d'acétate de potassium (0,41 g, 4,2 mmol) et d'acétate de palladium (0,69 g, 3,1 mmol) dans de l'alcool amylique anhydre (30 ml) est chauffé à 80 °C sous atmosphère d'argon pendant 2 h, puis filtré à chaud et concentré sous pression réduite. Le résidu est purifié par chromatographie sur colonne (SiO₂, méthanolldichlorométhane: 5/95) pour donner 370 mg (31 %) d'un solide blanc, p.f. > 275 °C.

RMN ¹H (DMSO): 0,73 (t, 3H); 1,21 (s, 9H); 1,86 (m, 2H); 2,77 (s, 3H); 2,77 (dd, 2H); 5,23 (s, 2H); 5,26 (s, 1H); 6,61 (s, 1H); 7,25 (s, 1H); 7,71 (t, 1H); 7,84 (t, 1H); 8,13 (d, 1H); 8,24 (d, 1H).

### Exemple 4 : 3-hydroxy-3-(4-oxo-4,6-dihydroindolizino [1,2-b] quinoléin-2-yl)pentanitrile :

### 4.a. 2-chloro-3-quinolylméthanol :

Une suspension de 2-chloro-3-quinoléinecarbaldéhyde (19,2 g, 100 mmol) dans du méthanol (400 ml) est traitée par du borohydrure de sodium (5,7 g, 150 mmol). Après 2 h d'agitation à température ambiante, le milieu réactionnel est concentré sous pression réduite puis repris à l'eau, filtré, lavé à l'eau et à l'éthanol pour donner, après séchage sous pression réduite, 15,8 g (81 %) d'un solide blanc, p.f. 166 °C.

RMN ¹H (DMSO): 4,70 (s, 2H); 5,96 (br, 1H); 7,5-8,2 (m, 4H); 8,48 (s, 1H).

### 4.b. 3-bromométhyl-2-chloroquinoléïne :

Une solution sous argon de 2-chloro-3-quinolylméthanol (obtenu selon **4.a**, 15,5 g 80 mmol) et de triphénylphosphine (32 g, 120 mmol) est traitée par de petites portions de tétrabromométhane (40 g, 120 mmol) en maintenant la température du milieu réactionnel inférieure à 30 °C. Aprés agitation sous argon pendant 2 h à température ambiante, le milieu réctionnel est concentré sous pression réduite, et le résidu est purifié par chromatographie sur colonne (SiO₂, acétate d'éthyle/heptane: 1/1) pour donner 13,5 g (66 %) d'un solide blanc, p.f. 125 °C.

RMN ¹H (DMSO) : 4,74 (s, 2H); 7,5-8,2 (m, 4H); 8,28 (s, 1H).

### 4.c. 4-(2-éthyl-1,3-dioxan-2-yl)-1,2-dihydro-2-pyridinone :

L'eau est distillée, de manière azéotropique pendant 16h avec un appareil de Dean-Stark, à partir d'un mélange de 1-(2-oxo-1,2-dihydro-4-pyridinyl)-1-propanone (obtenue selon **1.g,** 30 g, 198 mmol), d'éthylène glycol (60 ml) et d'acide *p*-toluènesulfonique (750 mg) dans le toluène (450 ml). Le solvant est ensuite évaporé sous pression réduite, le résidu est repris dans l'acétate d'éthyle (300 ml), lavé au bicarbonate de sodium aqueux saturé (100 ml) et à l'eau. La phase organique est séchée et concentrée. Le résidu est purifié par chromatographie sur colonne (SiO₂, méthanol/dichlorométhane: 5/95 à 7/93) pour donner 28 g (67 %) d'un solide blanc, p.f. 166 °C.

RMN ¹H (CDCl₃) : 0,86 (t, 3H); 1,32 (m, 1H); 1,69 (q, 2H); 2,10 (m, 1H); 3,82 (m, 4H); 6,35 (dd, 1H); 6,64 (d, 1H); 7,41 (d, 1H); 13,4 (br, 1H).

### 4.d. 1-(2-chloro-3-quinolylméthyl)-4-(2-éthyl-1,3-dioxan-2-yl)-1,2-dihydro-2-pyridinone :

Une solution sous argon de 4-(2-éthyl-1,3-dioxan-2-yl)-1,2-dihydro-2-pyridinone (obtenue selon **4.e,** 11 g, 52 mmol) dans du tétrahydrofurane anhydre (370 ml) est traitée à 0°C par de l'hydrure de sodium (80 % dans l'huile minérale, 1,68 g, 56 mmol). Le mélange résultant est maintenu sous agitation à 0 °C pendant 15 min puis traité par de la 3-bromométhyl-2-chloroquinoléïne (obtenu selon **4.b,** 13,4 g, 52 mmol) et le mélange résultant est maintenu sous agitation à température ambiante durant 24 h. Le milieu réactionnel est alors versé sur une solution aqueuse saturée en chlorure d'ammonium (400 ml), les phases sont séparées, et la phase aqueuse est extraite par du dichlorométhane. Les phases organiques combinées sont lavées au chlorure de sodium aqueux saturé, séchées sur sulfate de magnésium et concentrées sous pression réduite. Le résidu repris, à l'éther diéthylique, forme un précipité qui est recueilli par filtration et séché pour donner 13,8 g (69 %) d'un solide blanc.

RMN ¹H (CDCl₃) : 0,87 (t, 3H); 1,2-1,4 (m, 1H); 1,73 (q, 2H); 1,9-2,2 (m, 1H); 3,6-4,0 (m, 4H); 5,39 (s, 2H); 6,26 (d, 1H); 6,33 (d, 1H); 6,70 (d, 1H); 7,3-8,2 (m, 4H); 8,27 (s, 1H).

### 4.e. 1-(4-oxo-4,6-dihydroindolizino [1,2-b] quinoléin-2-yl)-1-propanone :

Un mélange de 1-(2-chloro-3-quinolylméthyl)-4-(2-éthyl-1,3-dioxan-2-yl)-1,2-dihydro-2-pyridinone (obtenue selon **4.d,** 13,8 g, 36 mmol), de bromure de tétrabutylammonium (23,2 g, 72 mmol), d'acétate de potassium (5,6 g, 72 mmol), de triphénylphosphine (3,77 g, 14 mmol) et d'acétate de palladium (1,57 g, 7 mmol) dans de l'acétonitrile anhydre (300 ml) est chauffé à 80 °C sous atmosphère d'argon pendant 16 h. Le milieu réactionnel est laissé à refroidir à température ambiante et le précipité est recueilli par filtration et lavé successivement à l'acétone, à l'eau, à l'acétonitrile et à l'éther diéthylique pour donner après séchage un solide blanc (6,2 g). Ce dernier est traité par de l'acide trifluoroacétique (60 ml) et de l'eau (20 ml) à température ambiante pendant 1 h. L'acide et l'eau sont chassés par distillation azéotropique au toluène et le résidu est suspendu dans de l'éther diéthylique pour donner après filtration et séchage 4,3 g (41 %) d'un solide blanc, p.f. > 275 °C.

RMN ¹H (CDCl₃) : 1,27 (t, 3H); 3,03 (q, 2H); 5, 28 (s, 2H); 7,19 (d, 1H); 7,6-8,0 (m, 4H); 8,23 (d, 1H); 8,36 (s, 1H).

### 4.f. 2-(2-éthyl-2-oxiranyl)-4,6-dihydroindolizino [1,2-b] quinoléin-4-one :

Une solution de 1-(4-oxo-4,6-dihydroindolizino [1,2-*b*] quinoléin-2-yl)-1-propanone (obtenue selon **4.e,** 1,74 g, 6 mmol) et d'iodure de triméthylsulfonium (2,45 g, 12 mmol) dans du diméthylsulfoxyde anhydre (30 ml) à 13 °C est placée sous atmosphère d'argon et traitée goutte à goutte par une solution de *tert*-butylate de potassium (1,34 g, 12 mmol) dans du diméthylsulfoxyde anhydre (8 ml). Le mélange résultant est agité pendant 15 min à température ambiante. Le milieu réactionnel est versé sur une solution aqueuse à 20 % d'acide acétique (50 ml) et extrait au dichlorométhane (2 x 50 ml). Les fractions organiques combinées sont lavées à l'eau et au chlorure de sodium aqueux saturé, séchées sur sulfate de magnésium, traitées au charbon actif, filtrées et concentrées sous pression réduite. Le résidu repris à l'éther diéthylique forme un précipité qui est recueilli par filtration et lavé à l'éther diéthylique pour donner après séchage 1,23 g (67 %) d'un solide blanc, p.f. 232 °C.

RMN ¹H (DMSO): 0,92 (t, 3H); 1,77 (m, 1H); 2,34 (m, 1H); 2,83 (d, 1H); 3,15 (d, 1H); 5,22 (s, 2H); 6,56 (s, 1H); 7,14 (s, 1H); 7,70 (t, 1H); 7,85 (t, 1H); 8,06 (d, 1H); 8,11 (d, 1H); 8,67 (s, 1H).

### 4. g. 3-hydroxy-3-(4-oxo-4,6-dihydroindolizino [1,2-b] quinoléin-2-yl)pentanitrile :

Une solution sous argon de chlorure de diéthylaluminium (solution molaire dans le dichlorométhane, 1,35 ml, 1,35 mmol) et de cyanure de triméthylsilyle (0,36 ml, 2,7 mmol) dans du dichlorométhane anhydre (12 ml) est traitée à 7 °C par une solution de 2-(2-éthyl-2-oxiranyl)-4,6-dihydroindolizino [1,2-*b*] quinoléin-4-one (obtenue selon **4.f,** 410 mg, 1,35 mmol) dans du dichlorométhane anhydre (26 ml) et le mélange résultant est agité pendant 48 h à température ambiante. Le milieu réactionnel est versé sur une solution aqueuse saturée en chlorure d'ammonium (50 ml) et extrait au dichlorométhane (2 x 25 ml). Les fractions organiques combinées sont lavées à l'eau et au chlorure de sodium aqueux saturé, séchées sur sulfate de magnésium, filtrées et concentrées sous pression réduite. Le résidu est purifié par chromatographie sur colonne (SiO₂, méthanol/dichlorométhane: 6/94) pour donner 122 mg (27 %) d'un solide blanc, p.f. 282 °C.

RMN ¹H (DMSO): 0,75 (t, 3H); 1,85 (m, 1H); 1,94 (m, 1H); 3,15 (dd, 1H); 5,23 (s, 2H); 5,91 (s, 1H); 6,70 (d, 1H); 7,35 (d, 1H); 7,70 (t, 1H); 7,86 (t, 1H); 8,14 (m, 2H); 8,67 (s, 1H).

### Exemple 5 : 3-hydroxy-3-(4-oxo-4,6-dihydroindolizino [1,2-b] quinoléin-2-yl)pentanoate de tert-butyle :

Une solution de diisopropylamine (0,79 ml, 6 mmol) dans du tétrahydrofurane anhydre (12 ml) est traitée goutte à goutte à 0 °C, sous argon, par du *n*-butyllithium (1,6 M dans l'hexane, 3,75 ml, 6 mmol). Le mélange résultant est agité à 0 °C pendant 15 min, puis refroidi à -78 °C et traité par de l'acétate de *tert*-butyle (0,81 ml, 6 mmol). Après agitation à -78 °C pendant 15 min, le réactif lithié résultant est traité goutte à goutte par une solution de 1-(4-oxo-4,6-dihydroindolizino [1,2-*b*] quinoléin-2-yl)-1-propanone (obtenu selon **4.e,** 870 mg, 3 mmol) dans du tétrahydrofurane anhydre (10 ml) et le mélange résultant est maintenu à -78 °C pendant 15 min, puis laissé remonter à 0 °C durant 1h. Le milieu réactionnel est hydrolysé par addition d'une solution aqueuse saturée en chlorure d'ammonium (60 ml) et le mélange résultant est extrait à l'acétate d'éthyle. Les phases organiques combinées sont lavées au chlorure de sodium aqueux saturé, séchées sur sulfate de magnésium et concentrées sous pression réduite. Le résidu est recristalisé dans l'alcool éthylique pour donner 800 mg (66 %) d'un solide blanc, p.f. 213 °C.

RMN ¹H (CDCl₃) : 0,86 (t, 3H); 1,37 (s, 9H); 1,85 (q, 2H); 2,85 (dd, 2H); 4,64 (s, 1H); 5,26 (s, 2H); 6,82 (s, 1H); 7,42 (s, 1H); 7,65 (t, 1H); 7,82 (t, 1H); 7,92 (d, 1H); 8,22 (d, 1H); 8,37 (s, 1H).

### Exemple 6 : acide 3-hydroxy-3-(4-oxo-4,6-dihydroindolizino [1,2-b] quinoléin-2-yl)pentanoïque :

Du 3-hydroxy-3-(4-oxo-4,6-dihydroindolizino [1,2-*b*] quinoléin-2-yl)pentanoate de *tert*-butyle (obtenu selon **5**, 750 mg, 1,85 mmol) est traité à l'acide trifluoroacétique (10 ml) pendant 1 h à température ambiante. Le milieu réactionnel est concentré sous pression réduite, repris au toluène et concentré de nouveau. Le résidu, repris à l'acétone (10 ml), forme un précipité qui est recueilli par filtration et lavé à l'acétone pour donner après séchage 450 mg (69 %) d'un solide blanc, p.f. 285 °C.

RMN ¹H (DMSO): 0,76 (t, 3H); 1,88 (m, 2H); 2,84 (dd, 2H); 5,22 (s, 2H); 6,62 (s, 1H); 7,29 (s, 1H); 7,70 (t, 1H); 7,85 (t, 1H); 8,11 (d, 1H); 8,15 (d, 1H); 8,66 (s, 1H); 12,1 (br, 1H).

### Exemple 7 : acide 3-(9-benzyloxy-10-fluoro-4-oxo-4,6-dihydroindolizino [1,2-b]quinoléin-2-yl)-3-hydroxypentanoïque :

On applique la procédure de l'exemple **6** en remplaçant le 3-hydroxy-3-(4-oxo-4,6-dihydroindolizino [1,2-*b*] quinoléin-2-yl)pentanoate de *tert*-butyle par du 3-(9-benzyloxy-10-fluoro-4-oxo-4,6-dihydroindolizino [1,2-*b*] quinoléin-2-yl)-3-hydroxypentanoate de *tert*-butyle (obtenu selon **1**). On obtient un solide jaune, p.f. 268 °C.

RMN ¹H (DMSO): 0,73 (t, 3H); 1,86 (m, 2H); 2,84 (dd, 2H); 5,20 (s, 2H); 5,30 (s, 1H); 5,37 (s, 2H); 6,60 (s, 1H); 7,22 (s, 1H); 7,3-7,7 (m, 5H); 7,87 (d, 1H); 7,95 (d, 1H); 8,55 (s, 1H); 12,14 (br, 1H).

### Exemple 8 : acide 3-(10-fluoro-9-méthoxy-4-oxo-4,6-dihydroindolizino [1,2-b] quinoléin-2-yl)-3-hydroxypentanoïque :

On applique la procédure de l'exemple **6** en remplaçant le 3-hydroxy-3-(4-oxo-4,6-dihydroindolizino [1,2-*b*] quinoléin-2-yl)pentanoate de *tert*-butyle par du 3-(10-fluoro-9-méthoxy-4-oxo-4,6-dihydroindolizino[1,2-*b*]quinoléin-2-yl)-3-hydroxy-pentanoate de *tert*-butyle obtenu selon **2**). On obtient un solide blanc; p.f. > 250 °C.

RMN ¹H (DMSO): 0,74 (t, 3H); 1,86 (m, 2H); 2,84 (dd, 2H); 4,03 (s, 3H); 5,19 (s, 2H); 5,29 (br, 1H); 6,35 (s, 1H); 7,22 (s, 1H); 7,74 (d, 1H); 7,92 (d, 1H); 8,56 (s, 1H); 12,1 (br, 1H).

### Exemple 9 : acide 3-hydroxy-3-(7-méthyl-4-oxo-4,6-dihydroindolizino [1,2-b] quinoléin-2-yl)pentanoïque :

On applique la procédure de l'exemple **6** en remplaçant le 3-hydroxy-3-(4-oxo-4,6-dihydroindolizino [1,2-*b*] quinoléin-2-yl)pentanoate de *tert*-butyle par du 3-hydroxy-3-(7-méthyl-4-oxo-4,6-dihydroindolizino [1,2-*b*] quinoléin-2-yl)pentanoate de *tert*-butyle (obtenu selon **3**). On obtient un solide blanc; p.f. > 250 °C.

RMN ¹H (DMSO): 0,73 (t, 3H); 1,86 (m, 2H); 2,77 (s, 3H); 2,84 (dd, 2H); 5,22 (s, 2H); 6,61 (s, 1H); 7,26 (s, 1H); 7,72 (t, 1H); 7,84 (t, 1H); 8,14 (d, 1H); 8,25 (d, 1H); 12,1 (br, 1H).

### Exemple 10 : acide 3-(9-benzyloxy-4-oxo-4,6-dihydroindolizino [1,2-b] quinoléin-2-yl)-3-hydroxypentanoïque :

On applique les procédures des exemples **1.i, 1.j** et **6** en remplaçant le 6-benzyloxy-7-fluoro-2-iodo-3-quinolylméthanol par du 6-benzyloxy-2-iodo-3-quinolylméthanol (obtenu selon la méthode décrite dans la demande de brevet PCT/FR96/00980). On obtient un solide blanc; p.f. 278 °C avec transition de phase à 180 °C.

RMN ¹H (DMSO) : 0,74 (t, 3H); 1,87 (m, 2H); 2,84 (dd, 2H); 5,19 (s, 2H); 5,29 (s, 3H); 6,59 (s, 1H); 7,22 (s, 1H); 7,3-7,7 (m, 7H); 8,07 (d, 1H); 8,51 (s, 1H); 12,13 (br, 1H).

### Exemple 11 : acide 3-(10-chloro-9-méthyl-4-oxo-4,6-dihydro-indolizino [1,2-b] quinoléin-2-yl)-3-hydroxypentanoïque :

On applique les procédures des exemples **1.a, 1.b, 1.e, 1.f, 1.i, 1.j** et **6** en partant de la 3-chloro-4-méthylaniline au lieu de la 3-fluoro-4-méthoxyaniline. On obtient un solide beige clair, p.f. > 250 °C.

RMN ¹H (DMSO): 0,73 (t, 3H); 1,86 (m, 2H); 2,53 (s, 3H); 2,84 (dd, 2H); 5,18 (s, 2H); 5,31 (br, 1H); 6,62 (s, 1H); 7,26 (s, 1H); 8,07 (s, 1H); 8,18 (s, 1H); 8,58 (s, 1H); 12,14 (br, 1H).

### Exemple 12 : 3-hydroxy-3-(4-oxo-4,6-dihydroindolizino [1,2-b] quinoléin-2-yl)pentanoate de méthyle :

Une solution d'acide 3-hydroxy-3-(4-oxo-4,6-dihydroindolizino [1,2-*b*] quinoléin-2-yl)-pentanoïque (obtenu selon la procédure de l'exemple **6,** 175 mg, 0,5 mmol) et d'iodure de méthyle (0,06 ml, 1 mmol) dans du diméthylsulfoxyde est traitée goutte à goutte par de la soude aqueuse (1N, 0,5 ml, 0,5 mmol) et le mélange résultant est agité 1 h à température ambiante. Le milieu réactionnel est versé sur de l'eau glacée (25 ml), extrait au dichlorométhane (2 x 25 ml), les fractions organiques combinées sont lavées au chlorure de sodium aqueux saturé, séchées sur sulfate de magnésium et concentrées sous pression réduite. Le résidu repris à l'éther diéthylique forme un précipité qui est recueilli par filtration et lavé à l'éther diéthylique pour donner après séchage 100 mg (55 %) d'un solide blanc, p.f. > 250 °C.

RMN ¹H (DMSO) : 0,74 (t, 3H); 1,89 (m, 2H); 2,93 (dd, 2H); 3,50 (s, 3H); 5,23 (s, 2H); 5,36 (s, 1H); 6,62 (s, 1H); 7,29 (s, 1H); 7,72 (t, 1H); 7,86 (t, 1H); 8,13 (m, 2H); 8,67 (s, 1H).

### Exemple 13 : 3-hydroxy-3-(4-oxo-4,6-dihydroindolizino [1,2-b] quinoléin-2-yl)pentanoate de tert-butylcarbonyloxyméthyle :

Une solution sous argon d'acide 3-hydroxy-3-(4-oxo-4,6-dihydroindolizino [1,2-*b*] quinoléin-2-yl)pentanoïque (obtenu selon **6,** 175 mg, 0,5 mmol) et de carbonate de potassium (100 mg, 0,75 mmol) dans du *N,N-*diméthylformamide (10 ml) est traitée goutte à goutte par de pivalate de chlorométhyle (0,14 ml, 1 mmol) et le mélange résultant est agité 24 h à température ambiante. Le milieu réactionnel est versé sur de l'eau (25 ml), extrait à l'acétate d'éthyle (3 x 25 ml), les fractions organiques combinées sont lavées au chlorure de sodium aqueux saturé, séchées sur sulfate de magnésium et concentrées sous pression réduite. Le résidu repris à l'éther diéthylique forme un précipité qui est recueilli par filtration et lavé à l'éther diéthylique pour donner après séchage 110 mg (47 %) d'un solide blanc, p.f. 192 °C.

RMN ¹H (DMSO): 0,74 (t, 3H); 1,02 (s, 9H); 1,88 (m, 2H); 2,99 (dd, 2H); 5,21 (s, 2H); 5,35 (s, 1H); 5,58 (dd, 2H), 6,62 (s, 1H); 7,30 (s, 1H); 7,70 (t, 1H); 7,85 (t, 1H); 8,11 (d, 1H); 8,15 (d, 1H); 8,67 (s, 1H).

### Exemple 14 : 2-[1-hydroxy-1-(1H-1,2,3,4-tétrazo-5-ylméthyl)propyl]-4,6-dihydroindolizino [1,2-b] quinoléin-4-one :

Une solution sous argon de 3-hydroxy-3-(4-oxo-4,6-dihydroindolizino [1,2-*b*] quinoléin-2-yl)pentanitrile (obtenu selon la procédure de l'exemple **4**, 70 mg, 0,21 mmol), d'azoture de triméthylsilyle (0,083 ml, 0,63 mmol) et d'une quantité catalytique d'oxyde de dibutylétain (5 mg) dans du toluène (5 ml) est chauffée au reflux pendant 2h. La masse résultante, qui a l'apparence d'une gomme, est traitée par de l'azoture de triméthylsilyle (1 ml) et du 1,2-dichloroéthane (2 ml). La solution obtenue est agitée pendant 1 h à température puis concentrée sous pression réduite. Le résidu, repris à l'éther diéthylique, forme un précipité qui est recueilli par filtration et purifié par chromatographie sur colonne (SiO₂, acide acétique/méthanol/dichlorométhane: 0,5/10/90) pour donner 30 mg (38 %) d'un solide blanc, p.f. > 250 °C.

RMN ¹H (DMSO): 0,79 (t, 3H); 1,87 (m, 1H); 1,99 (m, 1H); 3,42 (dd, 2H); 5,18 (s, 2H); 5,73 (br, 1H); 6,54 (d, 1H); 7,27 (d, 1H); 7,70 (t, 1H); 7,85 (t, 1H); 8,11 (d, 1H); 8,16 (d, 1H); 8,65 (s, 1H); 12,5 (br, 1H).

### Etude pharmacologique des produits de l'invention

### 1. Test d'inhibition de la relaxation de l'ADN surenroulé induite par la topoisomérase I.

De l'ADN plasmidique surenroulé (pUC 19, Pharmacia Biotech, Orsay, France, 300 ng) est incubé à 37°C pendant 15 min en présence de topoisomérase I de thymus de veau (Gibco-BRL, Paisley, Royaume-Uni, 1 unité) dans 20 µl de tampon de réaction (Tris-HCl pH 7.5: 50 mM, KCl: 50 mM, DTT: 0,5 mM, MgCl₂: 10 mM, EDTA: 0,1 mM, albumine de sérum bovin: 0,030 mg/ml) et d'un inhibiteur potentiel (préparé en solution extemporanée à 50 mM dans le diméthylsulfoxyde, puis dilué à l'eau distillée de manière à obtenir une concentration finale de 500 µM, 200 µM, 100 µM ou 10 µM, ne dépassant pas 1% de diméthylsulfoxyde). La réaction est stoppée par l'addition de 3 µl d'une solution dénaturante (protéinase K: 500 µg/ml, dodécylsulfate de sodium: 1 %, EDTA: 20 mM) suivie d'une incubation à 37°C durant 30 min, puis 2 µl d'un tampon de charge (bleu de bromophénol: 0,3 %, hydrogènephosphate de sodium: 10 mM, polysucrose Ficoll®-400: 16%) sont ajoutés et l'échantillon est déposé sur un gel d'agarose (Sea-Kem-GTG, FMC Bioproducts/Tebu, Perray-en-Yvelines, France) à 1,2 % contenant 2 µg/ml de chloroquine. La migration électrophorétique s'éffectue à une tension de 1V/cm pendant 20 h, avec recirculation du tampon d'électophorèse (Tris-HCl: 36 mM, dihydrogènephosphate de sodium: 30 mM, EDTA: 1 mM). Le gel est ensuite coloré sous agitation au bromure d'éthidium à 2 µg/ml, puis photographié sous lumière ultraviolette à 312 nm (caméra CCD Vilber-Lourmat, Lyon, France). Une analyse densitométrique (analyseur d'image BioProfil Vilber-Lourmat, Lyon, France) permet d'exprimer le pourcentage d'ADN relaxé par rapport à l'ADN total, à la concentration d'inhibiteur choisie. Les résultats sont consignés dans le tableau I ci-dessous pour le composé de formule générale (I) correspondant à l'exemple 6. Il apparaît qu'aux concentrations supérieures à 10 µM, le composé de l'exemple 6 est un meilleur inhibiteur de la topoisomérase I que la camptothécine, un inhibiteur connu de la topoisomérase I.

**TABLEAU I**

| **POURCENTAGE D'ADN RELAXÉ** | | | | |
|---|---|---|---|---|
| **Composé** | **CONCENTRATION (µM)** | | | |
| | **10** | **100** | **200** | **500** |
| Camptothécine | 95,5 ± 1,4 | 64,2 ± 6,1 | 60,6 ± 12,0 | 55,6 ± 8,8 |
| Exemple 6 | 95,2 ± 2,8 | 28,5 ± 3,5 | 15,5 ± 3,6 | 10,3 ± 2,8 |

### 2. Test d'inhibition de la relaxation de l'ADN surenroulé induite par la topoisomérase II.

De l'ADN plasmidique surenroulé (pUC 19, Pharmacia Biotech, Orsay, France, 300 ng) est incubé à 37°C pendant 15 min en présence de topoisomérase I de thymus de veau (Gibco-BRL, Paisley, Royaume-Uni, 7 unités) dans 20 µl de tampon de réaction (Tris-HCl pH 7.9: 10 mM, KCl: 50 mM, NaCl: 50 mM, MgCl₂: 5 mM, ATP: 1 mM, EDTA: 100 mM, albumine de sérum bovin: 15 mg/ml) et d'un inhibiteur potentiel (préparé en solution extemporanée à 50 mM dans le diméthylsulfoxyde, puis dilué à l'eau distillée de manière à obtenir une concentration finale de 500 µM, 200 µM, 100 µM ou 10 µM, ne dépassant pas 1% de diméthylsulfoxyde). La réaction est stoppée par l'addition de 3 µl d'une solution dénaturante (protéinase K: 500 µg/ml, dodécylsulfate de sodium: 1 %, EDTA: 20 mM) suivie d'une incubation à 37°C durant 30 min, puis 2 µl d'un tampon de charge (bleu de bromophénol: 0,3 %, hydrogènephosphate de sodium: 10 mM, polysucrose Ficoll® -400: 16%) sont ajoutés et l'échantillon est déposé sur un gel d'agarose (Sea-Kem-GTG, FMC Bioproducts/Tebu, Perray-en-Yvelines, France) à 1,2 % contenant 2 µg/ml de chloroquine. La migration électrophorétique s'éffectue à une tension de 1V/cm pendant 20 h, avec recirculation du tampon d'électophorèse (Tris-HCl: 36 mM, dihydrogènephosphate de sodium: 30 mM, EDTA: 1 mM). Le gel est ensuite coloré sous agitation au bromure d'éthidium à 2 µg/ml, puis photographié sous lumière ultraviolette à 312 nm (caméra CCD Vilber-Lourmat, Lyon, France). Une analyse densitométrique (analyseur d'image BioProfil Vilber-Lourmat, Lyon, France) permet d'exprimer le pourcentage d'ADN relaxé par rapport à l'ADN total, à la concentration d'inhibiteur choisie. Les résultats sont consignés dans le tableau I ci-dessous pour le composé de formule générale (I) correspondant à l'exemple 6. Il apparaît qu'aux concentrations supérieures à 10 µM, le composé de l'exemple 6 est un meilleur inhibiteur de la topoisomérase II que l'étoposide, un inhibiteur connu de la topoisomérase II.

**TABLEAU II**

| **POURCENTAGE D'ADN RELAXÉ** | | | | |
|---|---|---|---|---|
| **Composé** | **CONCENTRATION (µM)** | | | |
| | **10** | **100** | **200** | **500** |
| Etoposide | 92,6 ± 2,5 | 28,5 ±2,2 | 18,6 ± 1,4 | 12,0 ± 0,8 |
| Exemple 6 | 98,8 ± 0,4 | 9,9 ± 0,7 | 11,5 ± 1,9 | 6,5 ± 1,0 |

## Revendications

1. Un composé **caractérisé en ce que** ledit composé est de formule (I), 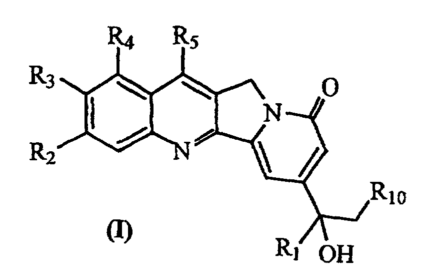 sous forme racémique, d'énantiomère ou toute combinaison de ces formes, dans laquelle
R₁ représente un radical alkyle, alkényle, alkynyle, haloalkyle, alkoxyalkyle ou alkylthioalkyle ;
R₂, R₃ et R₄ représentent, indépendamment, un radical H, hydroxy, alkoxy, arylalkoxy, halo, haloalkyle, alkyle, alkényle, cyano, cyanoalkyle, nitro, nitroalkyle, amido, amidoalkyle, (CH₂)ₘNR₆R₇, (CH₂)ₘOR₆, (CH₂)ₘSR₆, (CH₂)ₘCO₂R₆, (CH₂)ₘNR₆C(O)R₈, (CH₂)ₘC(O)R₈, (CH₂)ₘOC(O)R₈, O(CH₂)ₘNR₆R₇, OC(O)NR₆R₇, OC(O)(CH₂)ₘCO₂R₆ ou l'un des radicaux phényle, naphtyle, anthracyle, biphényle, indényle, phénylalkyle, naphtylalkyle, anthracylalkyle, biphénylalkyle ou indénylalkyle éventuellement substitués de une à quatre fois sur le noyau phényle, naphtyle, anthracyle, biphényle ou indényle par un ou des radicaux choisis parmi un radical alkyle, halo, nitro, amino, alkylamino, haloalkyle, hydroxyalkyle, alkoxy et alkoxyalkyle, ou R₂ et R₃, ou R₃ et R₄, ou R₄ et R₅, indépendamment forment ensemble une chaîne à 3 ou 4 chaînons, dans laquelle les éléments de la chaîne sont sélectionnés parmi le groupe constitué de CH, CH₂, O, S, N ou NR₉ ;
R₅ représente un radical H, halo, haloalkyle, alkyle, alkoxy, alkoxyalkyle, alkylthioalkyle, cycloalkyle, cycloalkylalkyle, cyano, cyanoalkyle, alcanesulphonylalkyle, hydroxyalkyle, nitro, (CH₂)ₘC(O)R₈, (CH₂)ₘNR₆C(O)R₈, (CH₂)ₘNR₆R₇, (CH₂)ₘN(CH₃)(CH₂)ₙNR₆R₇, (CH₂)ₘOC(O)R₈, (CH₂)ₘOC(O)NR₆R₇ ou l'un des radicaux phényle, naphtyle, anthracyle, biphényle, indényle, phénylalkyle, naphtylalkyle, anthracylalkyle, biphénylalkyle ou indénylalkyle éventuellement substitués de une à quatre fois sur le noyau phényle, naphtyle, anthracyle, biphényle ou indényle par un ou des radicaux choisis parmi un radical alkyle, halo, nitro, amino, alkylamino, haloalkyle, hydroxyalkyle, alkoxy et alkoxyalkyle ;
R₆ et R₇ représentent, indépendamment, H, un radical alkyle, hydroxyalkyle, alkylaminoalkyle, aminoalkyle, cycloalkyle, cycloalkylalkyle, alkényle, alkoxyalkyle, haloalkyle, l'un des radicaux phényle, naphtyle, anthracyle, biphényle, indényle, phénylalkyle, naphtylalkyle, anthracylalkyle, biphénylalkyle ou indénylalkyle éventuellement substitués de une à quatre fois sur le noyau phényle, naphtyle, anthracyle, biphényle ou indényle par un ou des radicaux choisis parmi un radical alkyle, halo, nitro, amino, alkylamino, haloalkyle, hydroxyalkyle, alkoxy et alkoxyalkyle, ou bien, lorsque les chaînes R₆ et R₇ sont rattachées à un même atome d'azote, R₆ et R₇ forment éventuellement un groupe morpholine, pipérazine ou pipéridine, ledit groupe étant éventuellement substitué par un ou plusieurs groupes choisis parmi les radicaux alkyle, phényle, naphtyle, anthracyle, biphényle, indényle, phénylalkyle, naphtylalkyle, anthracylalkyle, biphénylalkyle ou indénylalkyle, halo, nitro, amino, alkylamino, haloalkyle, hydroxyalkyle, alkoxy ou alkoxyalkyle ;
R₈ représente un radical H, alkyle, hydroxyalkyle, amino, alkylamino, alkylaminoalkyle, aminoalkyle, cycloalkyle, cycloalkylalkyle, alkényle, alkoxy, alkoxyalkyle, haloalkyle, ou l'un des radicaux phényle, naphtyle, anthracyle, biphényle, indényle, phénylalkyle, naphtylalkyle, anthracylalkyle, biphénylalkyle ou indénylalkyle éventuellement substitués de une à quatre fois sur le noyau phényle, naphtyle, anthracyle, biphényle ou indényle par un ou des groupes choisis parmi un radical alkyle, halo, nitro, amino, alkylamino, haloalkyle, hydroxyalkyle, alkoxy ou alkoxyalkyle ;
R₉ représente un radical H, alkyle, haloalkyle ou l'un des radicaux phényle, naphtyle, anthracyle, biphényle, indényle, phénylalkyle, naphtylalkyle, anthracylalkyle, biphénylalkyle ou indénylalkyle éventuellement substitués par un ou plusieurs groupes choisis parmi le radical alkyle, halo, nitro, amino, alkylamino, haloalkyle, hydroxyalkyle, alkoxy ou alkoxyalkyle ;
R₁₀ représente un radical cyano, C(O)OR₁₁, 1*H*-1,2,3,4-tétrazo-5-yle ou 1-alkyl-1,2,3,4-tétrazo-5-yle;
R₁₁ représente un radical H, alkyle, haloalkyle, hydroxyalkyle, alkylcarbonyloxyalkyle, (CH₂)ₚNR₆R₇ ou l'un des radicaux phényle, naphtyle, anthracyle, biphényle, indényle, phénylalkyle, naphtylalkyle, anthracylalkyle, biphénylalkyle ou indénylalkyle éventuellement substitués sur le cycle aromatique par un ou plusieurs groupes choisis parmi le radical alkyle, halo, nitro, amino, alkylamino, haloalkyle, hydroxyalkyle, alkoxy, ou alkoxyalkyle ;
m est un nombre entier compris entre 0 et 6 ;
n est un nombre entier compris entre 1 et 4 ;
p est un nombre entier compris entre 2 et 6 ;
étant entendu que :
- chacun des groupes alkyle entrant dans les définitions des radicaux ci-dessus est linéaire ou ramifié et comporte de 1 à 6 atomes de carbone ;
- chacun des groupes alkényle ou alkynyle entrant dans les définitions des radicaux ci-dessus est linéaire ou ramifié et comporte de 2 à 6 atomes de carbone ; et
- chacun des groupes cycloalkyle entrant dans les définitions des radicaux ci-dessus comporte de 3 à 7 atomes de carbone ;
ou un sel pharmaceutiquement acceptable de ce dernier.

2. Un composé selon la revendication 1, **caractérisé en ce que**
R₁ représente un radical alkylé ;
R₂ représente un atome d'hydrogène ou d'halogène ;
R₃ représente un atome d'hydrogène ou d'halogène, ou un radical alkylé ou OR₆ dans lequel R₆ représente H, un radical alkyle, phénylalkyle, naphtylalkyle, anthracylalkyle, biphénylalkyle ou indénylalkyle ;
R₄ représente un atome d'hydrogène;
R₅ représente un atome d'hydrogène ou un radical alkyle ;
R₁₀ représente un radical cyano, C(O)OR₁₁ ou 1H-1,2,3,4-tétrazo-5-yle ;
R₁₁ représente un radical H, alkyle, haloalkyle, hydroxyalkyle, alkylcarbonyloxyalkyle, (CH₂)ₚNR₆R₇ ou l'un des radicaux phényle, naphtyle, anthracyle, biphényle, indényle, phénylalkyle, naphtylalkyle, anthracylalkyle, biphénylalkyle ou indénylalkyle éventuellement substitués sur le cycle aromatique par un ou plusieurs groupes choisis parmi le radical alkyle, halo, nitro, amino, alkylamino, haloalkyle, hydroxyalkyle, alkoxy ou alkoxyalkyle ;
ou un sel pharmaceutiquement acceptable de ce dernier.

3. Un composé selon la revendication 2, **caractérisé en ce que**
R₁ représente un groupe éthyle;
R₂ représente un atome d'hydrogène, de chlore ou de fluor;
R₃ représente un atome d'hydrogène, de chlore ou de fluor, ou un radical méthyle, méthoxy ou benzyloxy;
R₄ représente un atome d'hydrogène;
R₅ représente un atome d'hydrogène ou un radical alkyle ;
R₁₀ représente un radical cyano, C(O)OR₁₁ ou 1H-1,2,3,4-tétrazo-5-yle ;
R₁₁ représente un radical H, alkyle, haloalkyle, hydroxyalkyle, alkylcarbonyloxyalkyle, (CH₂)ₚNR₆R₇ ou l'un des radicaux phényle, naphtyle, anthracyle, biphényle, indényle, phénylalkyle, naphtylalkyle, anthracylalkyle, biphénylalkyle ou indénylalkyle éventuellement substitués sur le cycle aromatique par un ou plusieurs groupes choisis parmi le radical alkyle, halo, nitro, amino, alkylamino, haloalkyle, hydroxyalkyle, alkoxy, ou alkoxyalkyle ;
ou un sel pharmaceutiquement acceptable de ce dernier.

4. Un composé selon l'une des revendications 1 à 3, **caractérisé en ce** ledit composé est choisi parmi les composés répondant aux formules suivantes :
- 3-(9-benzyloxy-10-fluoro-4-oxo-4,6-dihydroindolizino [1,2-*b*]quinoléin-2-yl)-3-hydroxypentanoate de *tert*-butyle;
- 3-(10-fluoro-9-méthoxy-4-oxo-4,6-dihydroindolizino [1,2-*b*] quinoléin-2-yl)-3-hydroxypentanoate de *tert*-butyle;
- 3-hydroxy-3-(7-méthyl-4-oxo-4,6-dihydroindolizino [1,2-*b*] quinoléin-2-yl)pentanoate de *tert*-butyle;
- 3-hydroxy-3-(4-oxo-4,6-dihydroindolizino [1,2-*b*] quinoléin-2-yl)pentanitrile;
- 3-hydroxy-3-(4-oxo-4,6-dihydroindolizino [1,2-*b*] quinoléin-2-yl)pentanoate de *tert*-butyle;
- acide 3-hydroxy-3-(4-oxo-4,6-dihydroindolizino [1,2-*b*] quinoléin-2-yl)pentanoïque;
- acide 3-(9-benzyloxy-10-fluoro-4-oxo-4,6-dihydroindolizino [1,2-*b*]quinoléin-2-yl)-3-hydroxypentanoïque;
- l'acide 3-(10-fluoro-9-méthoxy-4-oxo-4,6-dihydro-indolizino [1,2-*b*] quinoléin-2-yl)-3-hydroxypentanoïque;
- acide 3-hydroxy-3-(7-méthyl-4-oxo-4,6-dihydroindolizino [1,2-*b*] quinoléin-2-yl)pentanoïque;
- acide 3-(9-benzyloxy-4-oxo-4,6-dihydroindolizino [1,2-*b*] quinoléin-2-yl)-3-hydroxypentanoïque;
- acide 3-(10-chloro-9-méthyl-4-oxo-4,6-dihydro-indolizino [1,2-*b*] quinoléin-2-yl)-3-hydroxypentanoïque;
- 3-hydroxy-3-(4-oxo-4,6-dihydroindolizino [1,2-*b*] quinoléin-2-yl)pentanoate de méthyle;
- 3-hydroxy-3-(4-oxo-4,6-dihydroindolizino [1,2-*b*] quinoléin-2-yl)pentanoate de *tert*-butylcarbonyloxyméthyle;
- 2-[1-hydroxy-1-(1H-1,2,3,4-tétrazo-5-ylméthyl)propyl]-4,6-dihydroindolizino [1,2-*b*] quinoléin-4-one;
ou un sel pharmaceutiquement acceptable de ces derniers.

5. Un composé selon l'une des revendications 1 à 4, **caractérisé en ce qu'**il s'agit de l'acide 3-hydroxy-3-(4-oxo-4,6-dihydroindolizino [1,2-*b*] quinoléin-2-yl)pentanoïque ou d'un sel pharmaceutiquement acceptable de ce dernier.

6. Un procédé de préparation des composés de formule générale (I) selon l'une quelconque des revendications 1 à 5, **caractérisé en ce que**
- l'on *N*-alkyle une pyridinone de formule générale A 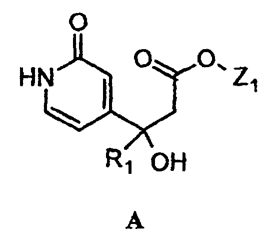
dans laquelle R₁ a la signification indiquée dans la revendication 1 et le groupe Z₁ représente un radical alkyle linéaire ou ramifié comptant de 1 à 6 atomes de carbone, avec une quinoléine de formule générale B 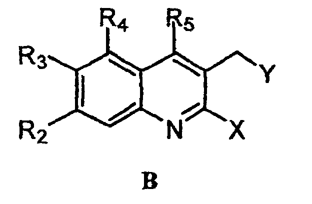 dans laquelle R₂, R₃, R₄ et R₅ ont la signification indiquée dans la revendication 1, X représente un atome de chlore, de brome ou d'iode et Y représente soit un atome de brome, soit une fonction hydroxyle,
pour donner le composé de formule générale C 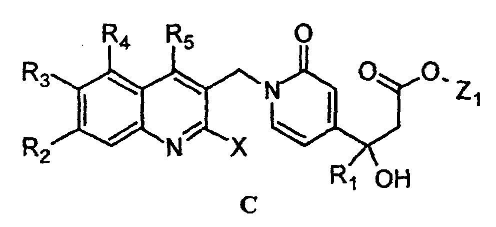 dans lequel R₁, R₂, R₃, R₄, R₅, X, et Z₁ ont la signification indiquée dans la revendication 1,
- puis l'on cyclise le composé de formule générale C pour obtenir le composé de formule générale (I) telle que définie dans la revendication 1, dans laquelle R₁₀ représente un radical carbalkoxy.

7. Un procédé de préparation d'un composé de formule générale (I) **caractérisé en ce que**
- l'on *N*-alkyle une pyridinone de formule générale D 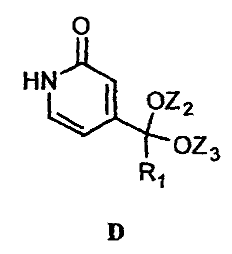
dans laquelle R₁ a la signification indiquée dans la revendication 1 et les groupes Z₂ et Z₃ représentent, indépendamment, un alkyle linéaire ou ramifié comptant de 1 à 6 atomes de carbone ou Z₂ et Z₃ forment ensemble une chaîne hydrocarbonée saturée de 2 à 4 carbones, avec une quinoléine de formule générale B telle que définie dans la revendication 6 pour donner le composé de formule générale E 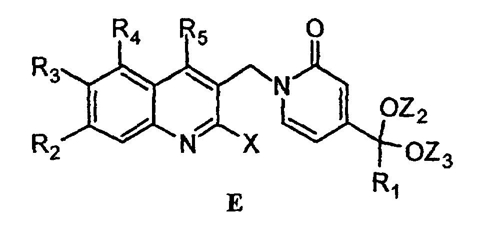 dans laquelle R₁, R₂, R₃, R₄, R₅, X, Z₂ et Z₃ ont la signification indiquée dans la revendication 1,
- puis l'on cyclise le composé de formule générale E pour obtenir le composé de formule générale F 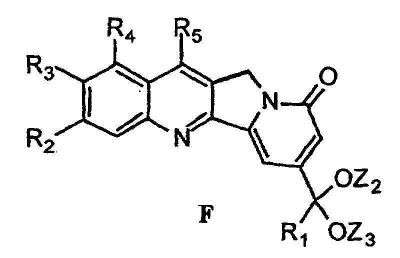
dans laquelle R₁, R₂, R₃, R₄, R₅, Z₂ et Z₃ ont la signification indiquée dans la revendication 1,
- puis on libère la fonction carbonyle protégée du composés de formule générale F pour donner le composé de formule générale G 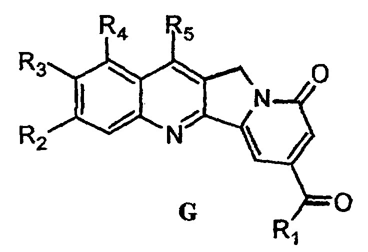
dans laquelle R₁, R₂, R₃, R₄, et R₅ ont la signification indiquée dans la revendication 1,
- puis la fonction carbonyle du composé de formule générale G est traitée par un agent époxydant pour donner le composé de formule générale H 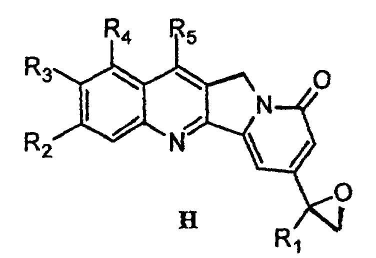
dans laquelle R₁, R₂, R₃, R₄, et R₅ ont la signification indiquée dans la revendication 1,
puis l'époxyde du composé de formule générale H est traité un par un agent nitrilant pour donner le composé de formule générale (I) telle que définie dans la revendication I, dans laquelle R₁₀ représente un radical cyano.

8. Un procédé de préparation d'un composés de formule générale (I) selon l'une des revendications 1 à 5, **caractérisé en ce que** fonction carbonyle d'un composé de formule générale G tel que définie dans la revendication 7 est traitée par un agent alkylant approprié pour donner le composé de formule générale (I) correspondant.

9. Un procédé de préparation d'un composé de formule générale (I) selon l'une des revendications 1 à 5, **caractérisé en ce que** la fonction ester d'un composé de formule générale (I) dans laquelle R₁₀ représente un radical carbalkoxy est saponifiée pour donner un composé de formule générale (I) dans laquelle R₁₀ représente un radical carboxy.

10. Un procédé de préparation des composés de formule générale (I) selon l'une des revendications 1 à 5, **caractérisé en ce que** fonction acide d'un composé de formule générale (I) dans laquelle R₁₀ représente un radical carboxy est estérifiée pour donner un composé de formule générale (I) dans laquelle R₁₀ représente un radical carbalkoxy.

11. Un procédé de préparation des composés de formule générale (I) selon l'une des revendications 1 à 5 **caractérisé en ce que** la fonction ester d'un composé de formule générale (I) dans laquelle R₁₀ représente un radical carbalkoxy est transestérifié pour donner un composé de formule générale (I) dans laquelle R₁₀ représente un autre radical carbalkoxy.

12. Un procédé de préparation des composés de formule générale (I) **caractérisé en ce que** la fonction nitrile d'un composé de formule générale (I) dans laquelle R₁₀ représente un radical cyano subi une addition dipolaire avec un azoture pour donner un composé de formule générale (I) dans laquelle R₁₀ représente un radical 1,2,3,4-tétrazole-5-yle.

13. A titre de produits industriels nouveaux, les composés de formule générale A 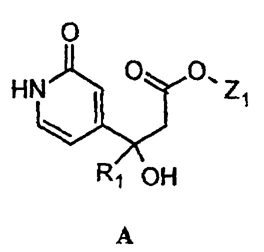 dans laquelle R₁ représente un radical alkyle, alkényle, alkynyle, haloalkyle, alkoxyalkyle ou alkylthioalkyle, et le groupe Z₁ représente un radical alkyle ;
étant entendu que :
- chacun des groupes alkyle entrant dans les définitions des radicaux ci-dessus est linéaire ou ramifié et comporte de 1 à 6 atomes de carbone ; et
- chacun des groupes alkényle ou alkynyle entrant dans les définitions des radicaux ci-dessus est linéaire ou ramifié et comporte de 2 à 6 atomes de carbone.

14. Un procédé de préparation des composés de formule générale A selon la revendication 13, **caractérisé en ce que** l'on déprotège une 2-alkoxypyridine de formule générale J 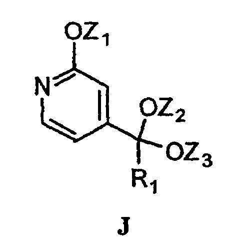 dans laquelle R₁ représente un radical alkyle, alkényle, alkynyle, haloalkyle, alkoxyalkyle ou alkylthioalkyle, le groupe Z₁ représente un radical alkyle et les groupes Z₂ et Z₃ représentent, indépendamment, un radical alkyle ou Z₂ et Z₃ forment ensemble une chaîne hydrocarbonée saturée de 2 à 4 carbones, pour donner la pyridinone de formule générale K 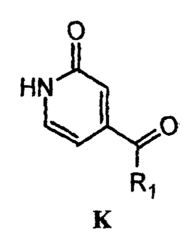 dans laquelle R₁ a la signification indiquée dans la revendication 1;
puis l'on traite par un agent alkylant fonctionnalisé le composé de formule générale K pour obtenir un composé de formule générale A dans laquelle R₁ et Z₁ ont la signification indiquée dans la revendication 13 ;
étant entendu que :
- chacun des groupes alkyle entrant dans les définitions des radicaux ci-dessus est linéaire ou ramifié et comporte de 1 à 6 atomes de carbone ; et
- chacun des groupes alkényle ou alkynyle entrant dans les définitions des radicaux ci-dessus est linéaire ou ramifié et comporte de 2 à 6 atomes de carbone.

15. A titre de produits industriels nouveaux, les composés de formule générale D 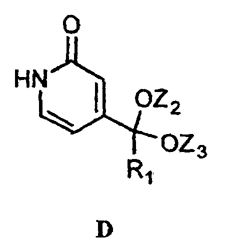 dans laquelle R₁ représente un radical alkyle, alkényle, alkynyle, haloalkyle, alkoxyalkyle ou alkylthioalkyle, et les groupes Z₂ et Z₃ représentent, indépendamment, un radical alkyle ou Z₂ et Z₃ forment ensemble une chaîne hydrocarbonée saturée de 2 à 4 carbones ;
étant entendu que :
- chacun des groupes alkyle entrant dans les définitions des radicaux ci-dessus est linéaire ou ramifié et comporte de 1 à 6 atomes de carbone ; et
- chacun des groupes alkényle ou alkynyle entrant dans les définitions des radicaux ci-dessus est linéaire ou ramifié et comporte de 2 à 6 atomes de carbone.

16. Un procédé de préparation des composés de formule générale D selon la revendication 15, **caractérisé en ce que** l'on protège la fonction cétonique dans le composé de formule générale K tel que défini dans la revendication 14 pour obtenir un composé de formule générale D dans laquelle R₁, Z₂ et Z₃ ont la signification indiquée dans la revendication 15.

17. A titre de produits industriels nouveaux, les composés de formule générale K 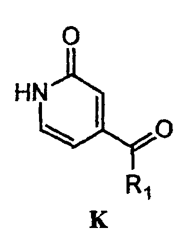 dans laquelle R₁ représente un radical alkyle, alkényle, alkynyle, haloalkyle, alkoxyalkyle ou alkylthioalkyle ;
étant entendu que :
- chacun des groupes alkyle entrant dans les définitions des radicaux ci-dessus est linéaire ou ramifié et comporte de 1 à 6 atomes de carbone ; et
- chacun des groupes alkényle ou alkynyle entrant dans les définitions des radicaux ci-dessus est linéaire ou ramifié et comporte de 2 à 6 atomes de carbone.

18. A titre de médicament, un composé selon l'une quelconque des revendications 1 à 5, ou un sel pharmaceutiquement acceptable de ce dernier.

19. Composition pharmaceutique contenant, à titre de principe actif, l'un au moins des composés selon l'une quelconque des revendications 1 à 5.

20. Utilisation d'un composé selon l'une quelconque des revendications 1 à 5 pour la préparation de médicaments destinés à inhiber les topoisomérases de type I, ou les topoisomérases de type II, ou, simultanément, les deux types de topoisomérases.

21. Utilisation d'un composé selon l'une quelconque des revendications 1 à 5 pour la préparation de médicaments antitumoraux.

22. Utilisation d'un composé selon l'une quelconque des revendications 1 à 5 pour la préparation de médicaments antiviraux.

23. Utilisation d'un composé selon l'une quelconque des revendications 1 à 5 pour la préparation de médicaments antiparasitaires.

## Patentansprüche

1. Verbindung, **dadurch gekennzeichnet, dass** sie der Formel (I) 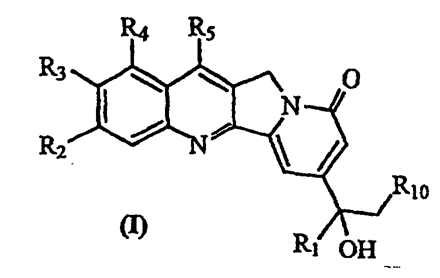 in Form von Racemat, in Form von Enantiomer oder in jeder Kombination dieser Formen entspricht, in der
R₁ einen Alkyl-, Alkenyl-, Alkinyl-, Haloalkyl-, Alkoxyalkyl- oder Alkylthioalkylrest darstellt;
R₂, R₃ und R₄ unabhängig voneinander einen der Reste H, Hydroxy, Alkoxy, Arylalkoxy, Halogen, Halogenalkyl, Alkyl, Alkenyl, Cyano, Cyanoalkyl, Nitro, Nitroalkyl, Amido, Amidoalkyl, (CH₂)ₘNR₆R₇, (CH₂)ₘOR₆, (CH₂)ₘSR₆, (CH₂)ₘCO₂R₆, (CH₂)ₘNR₆C(O)R₈, (CH₂)ₘC(O)R₈, (CH₂)ₘOC(O)R₈, O (CH₂)ₘNR₆R₇, OC(O)NR₆R₇, OC(O)(CH₂)ₘCO₂R₆ oder einen der Reste Phenyl, Naphtyl, Anthracyl, Biphenyl, Indenyl, Phenylalkyl, Naphtylalkyl, Anthracylalkyl, Biphenylalkyl oder Indenylalkyl darstellen, die ggf. ein- bis viermal an dem Phenyl-, Naphtyl-, Anthracyl-, Biphenyl- oder Indenylring durch einen oder mehrere Reste substituiert sind, die aus einem Alkyl-, Halogen-, Nitro-, Amino-, Alkylamino-, Halogenalkyl-, Hydroxyalkyl-, Alkoxy- und Alkoxyalkylrest ausgewählt sind, worin R₂ und R₃, oder R₃ und R₄, oder R₄ und R₅ unabhängig voneinander zusammen eine Kette mit 3 oder 4 Gliedern bilden, in der die Elemente der Kette aus der aus CH, CH₂, O, S, N oder NR₉ bestehenden Gruppe ausgewählt sind;
R₅ einen der Reste H, Halogen, Halogenalkyl, Alkyl, Alkoxy, Alkoxyalkyl, Alkylthioalkyl, Cycloalkyl, Cycloalkylalkyl, Cyano, Cyanoalkyl, Alkansulfonylalkyl, Hydroxyalkyl, Nitro, (CH₂)ₘC(O)R₈, (CH₂)ₘNR₆C(O)R₈, (CH₂)ₘNR₆R₇, (CH₂)ₘN(CH₃) (CH₂)ₙNR₆R₇, (CH₂)ₘOC(O)R₈, (CH₂)ₘOC(O)NR₆R₇ oder einen der Reste Phenyl, Naphtyl, Anthracyl, Biphenyl, Indenyl, Phenylalkyl, Naphtylalkyl, Anthracylalkyl, Biphenylalkyl oder Indenylalkyl darstellt, die gegebenenfalls ein- bis viermal an dem Phenyl-, Naphtyl-, Anthracyl-, Biphenyl- oder Indenylring durch einen oder mehrere Reste substituiert sind, die aus einem Alkyl-, Halogen-, Nitro-, Amino-, Alkylamino-, Halogenalkyl-, Hydroxyalkyl-, Alkoxy- und Alkoxyalkylrest ausgewählt sind;
R₆ und R₇ unabhängig voneinander H, einen Alkyl-, Hydroxyalkyl-, Alkylaminoalkyl-, Aminoalkyl-, Cycloalkyl-, Cycloalkylalkyl-, Alkenyl-, Alkoxyalkyl-, Halogenalkylrest, einen der Reste Phenyl, Naphtyl, Anthracyl, Biphenyl, Indenyl, Phenylalkyl, Naphtylalkyl, Anthracylalkyl, Biphenylalkyl oder Indenylalkyl darstellen, die gegebenenfalls ein- bis viermal an dem Phenyl-, Naphtyl-, Anthracyl-, Biphenyl- oder Indenylring durch einen oder mehrere Reste substituiert sind, die aus einem Alkyl-, Halogen-, Nitro-, Amino-, Alkylamino-,Halogenalkyl-, Hydroxyalkyl-, Alkoxy- und Alkoxyalkylrest ausgewählt sind, oder, wenn die Ketten R₆ und R₇ am selben Stickstoffatom befestigt sind, R₆ und R₇ ggf. eine Morpholin-, Piperazin- oder Piperidingruppe bilden, wobei diese Gruppe ggf. durch eine oder mehrere Gruppen substituiert ist, die aus den Resten Alkyl, Phenyl, Naphtyl, Anthracyl, Biphenyl, Indenyl, Phenylalkyl, Naphtylalkyl, Anthracylalkyl, Biphenylalkyl oder Indenylalkyl, Halogen, Nitro, Amino, Alkylamino, Halogenalkyl, Hydroxyalkyl, Alkoxy oder Alkoxyalkyl ausgewählt sind;
R₈ einen H-, Alkyl-, Hydroxyalkyl-, Amino-, Alkylamino-, Alkylaminoalkyl-, Aminoalkyl-, Cycloalkyl-, Cycloalkylalkyl-, Alkenyl-, Alkoxy-, Alkoxyalkyl-, Halogenalkylrest oder einen der Reste Phenyl, Naphtyl, Anthracyl, Biphenyl, Indenyl, Phenylalkyl, Naphtylalkyl, Anthracylalkyl, Biphenylalkyl oder Indenylalkyl darstellt, die ggf. ein- bis viermal an dem Phenyl-, Naphtyl-, Anthracyl-, Biphenyl- oder Indenylring durch eine oder mehrere Gruppen substituiert sind, die aus einem Alkyl-, Halogen-, Nitro-, Amino-, Alkylamino-, Halogenalkyl-, Hydroxyalkyl-, Alkoxy- oder Alkoxyalkylrest ausgewählt sind;
R₉ einen H-, Alkyl-, Halogenalkylrest oder einen der Reste Phenyl, Naphtyl, Anthracyl, Biphenyl, Indenyl, Phenylalkyl, Naphtylalkyl, Anthracylalkyl, Biphenylalkyl oder Indenylalkyl darstellt, die ggf. durch eine oder mehrere Gruppen substituiert sind, die aus einem Alkyl-, Halogen-, Nitro-, Amino-, Alkylamino-, Halogenalkyl-, Hydroxyalkyl-, Alkoxy- oder Alkoxyalkylrest ausgewählt sind;
R₁₀ einen der Reste Cyano, C(O)OR₁₁, 1*H*-1,2,3,4-Tetrazo-5-yl oder 1-Alkyl-1,2,3,4-tetrazo-5-yl darstellt;
R₁₁ einen der Reste H, Alkyl, Halogenalkyl, Hydroxyalkyl, Alkylcarbonyloxyalkyl, (CH₂)ₚNR₆R₇ oder einen der Reste Phenyl, Naphtyl, Anthracyl, Biphenyl, Indenyl, Phenylalkyl, Naphtylalkyl, Anthracylalkyl, Biphenylalkyl oder Indenylalkyl darstellt, die ggf. an dem aromatischen Ring durch eine oder mehrere Gruppen substituiert sind, die aus einem Alkyl-, Halogen-, Nitro-, Amino-, Alkylamino-, Halogenalkyl-, Hydroxyalkyl-, Alkoxy- oder Alkoxyalkylrest ausgewählt sind;
m eine ganze Zahl zwischen 0 und 6 ist;
n eine ganze Zahl zwischen 1 und 4 ist;
p eine ganze Zahl zwischen 2 und 6 ist;
wobei
- jede der in den Definitionen der oben genannten Reste auftretenden Alkylgruppen verzweigt oder unverzweigt ist und 1 bis 6 Kohlenstoffatome besitzt,
- jede der in den Definitionen der oben genannten Reste auftretenden Alkenyl- oder Alkinylgruppen verzweigt oder unverzweigt ist und 2 bis 6 Kohlenstoffatome besitzt und
- jede der in den Definitionen der oben genannten Reste auftretenden Cycloalkylgruppen 3 bis 7 Kohlenstoffatome besitzt;
oder ein pharmazeutisch verträgliches Salz dieser Verbindung.

2. Verbindung nach Anspruch 1, **dadurch gekennzeichnet, dass**
R₁ einen Alkylrest darstellt;
R₂ ein Wasserstoff- oder Halogenatom darstellt;
R₃ ein Wasserstoff- oder Halogenatom oder einen Alkylrest oder den Rest OR₆ darstellt, in dem R₆ für H, einen Alkyl-, Phenylalkyl-, Naphtylalkyl- Anthracylalkyl-, Biphenylalkyl- oder Indenylalkylrest steht;
R₄ ein Wasserstoffatom darstellt;
R₅ ein Wasserstoffatom oder einen Alkylrest darstellt;
R₁₀ einen der Reste Cyano, C(O)OR₁₁ oder 1H-1,2,3,4-Tetrazo-5-yl darstellt;
R₁₁ einen der Reste H, Alkyl, Halogenalkyl, Hydroxyalkyl, Alkylcarbonyloxyalkyl, (CH₂)ₚNR₆R₇ oder einen der Reste Phenyl, Naphtyl, Anthracyl, Biphenyl, Indenyl, Phenylalkyl, Naphtylalkyl, Anathracylalkyl, Biphenylalkyl oder Indenylalkyl darstellt, die ggf. an dem aromatischen Ring durch eine oder mehrere Gruppen substituiert sind, die aus einem Alkyl-, Halogen-, Nitro-, Amino-, Alkylamino-, Halogenalkyl-, Hydroxyalkyl-, Alkoxy- oder Alkoxyalkylrest ausgewählt sind; oder ein pharmazeutisch verträgliches Salz dieser Verbindung.

3. Verbindung nach Anspruch 2, **dadurch gekennzeichnet, dass**
R₁ eine Ethylgruppe darstellt;
R₂ ein Wasserstoff-, Chlor- oder Fluoratom darstellt;
R₃ ein Wasserstoff-, Chlor- oder Fluoratom oder einen Methyl-, Methoxy- oder Benzyloxyrest darstellt;
R₄ ein Wasserstoffatom darstellt;
R₅ ein Wasserstoffatom oder einen Alkylrest darstellt;
R₁₀ einen der Reste Cyano, C(O)OR₁₁ oder 1H-1,2,3,4-Tetrazo-5-yl darstellt;
R₁₁ einen der Reste H, Alkyl, Halogenalkyl Hydroxyalkyl, Alkylcarbonyloxyalkyl, (CH₂)ₚNR₆R₇ oder einen der Reste Phenyl, Naphtyl, Anthracyl, Biphenyl, Indenyl, Phenylalkyl, Naphtylalkyl, Anthracylalkyl, Biphenylalkyl oder Indenylalkyl darstellt, die ggf. an dem aromatischen Ring durch eine oder mehrere Gruppen substituiert sind, die aus einem Alkyl-, Halogen-, Nitro-, Amino-, Alkylamino-, Halogenalkyl-, Hydroxyalkyl-, Alkoxy- oder Alkoxyalkylrest ausgewählt sind; oder ein pharmazeutisch verträgliches Salz dieser Verbindung.

4. Verbindung nach einem der Ansprüche 1 bis 3, **dadurch gekennzeichnet, dass** diese Verbindung aus den Verbindungen ausgewählt ist, die den folgenden allgemeinen Formeln entsprechen:
- 3-(9-Benzyloxy-10-fluor-4-oxo-4,6-dihydroindolizino[1,2-*b*]chinolin-2-yl)-3-hydroxy-*tert.*-butylpentanoat;
- 3-(10-Fluor-9-methoxy-4-oxo-4,6-dihydroindolizino[1,2-*b*]chinolin-2-yl)-3-hydroxy- *tert.*-butylpentanoat;
- 3-Hydroxy-3-(7-methyl-4-oxo-4,6-dihydroindolizino[1,2-*b*]chinolin-2-yl) *tert. -* butylpentanoat;
- 3-Hydroxy-3-(4-oxo-4,6-dihydroindolizino[1,2-*b*]chinolin-2-yl)pentanitril;
- 3-Hydroxy-3-(4-oxo-4,6-dihdroindolizino[1,2-*b*]chinolin-2-yl)*tert.*butylpentanoat;
- 3-Hydroxy-3-(4-oxo-4,6-dihydroindolizino[1,2-*b*]chinolin-2-yl)pentansäure;
- 3-(9-Benzyloxy-10-fluor-4-oxo-4,6-dihydroindolizino[1,2-*b*]chinolin-2-yl)-3-hydroxypentansäure;
- 3-(10-Fluor-9-methoxy-4-oxo-4,6-dihydroindolizino[1,2-*b*]chinolin-2-yl)-3-hydroxypentansäure;
- 3-Hydroxy-3-(7-methyl-4-oxo-4,6-dihydroindolizino[1,2-*b*]chinolin-2-yl)pentansäure;
- 3-(9-Benzyloxy-4-oxo-4,6-dihydroindolizino[1,2-*b*]chinolin-2-yl)-3-hydroxypentansäure;
- 3-(10-Chlor-9-methyl-4-oxo-4,6-dihydroindolizino[1,2-*b*]chinolin-2-yl)-3-hydroxypentansäure;
- 3-Hydroxy-3-(4-oxo-4,6-dihydroindolizino[1,2-*b*]chinolin-2-yl)methylpentanoat;
- 3-Hydroxy-3-(4-oxo-4,6-dihydroindolizino[1,2-*b*]chinolin-2-yl)*tert.*butylcarbonyloxymethylpentanoat;
- 2-[1-Hydroxy-1-(1H-1,2,3,4-tetrazo-5-ylmethyl)propyl]-4,6-dihydroindolizino[1,2-*b*]chinolin-4-on;
oder ein pharmazeutisch verträgliches Salz dieser Verbindungen.

5. Verbindung nach einem der Ansprüche 1 bis 4, **dadurch gekennzeichnet, dass** es sich um 3-Hydroxy-3-(4-oxo-4,6-dihydroindolizino[1,2-*b*]chinolin-2-yl)pentansäure oder ein pharmazeutisch verträgliches Salz dieser Säure handelt.

6. Verfahren zur Herstellung der Verbindungen der allgemeinen Formel (I) nach einem der Ansprüche 1 bis 5, **dadurch gekennzeichnet, dass** man
ein Pyridinon der allgemeinen Formel A 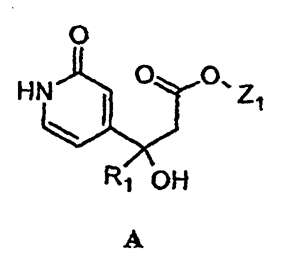 in der R₁ die in Anspruch 1 angegebene Bedeutung hat und die Gruppe Z₁ einen verzweigten oder unverzweigten Alkylrest mit 1 bis 6 Kohlenstoffatomen darstellt, mit einem Chinolin der allgemeinen Formel B 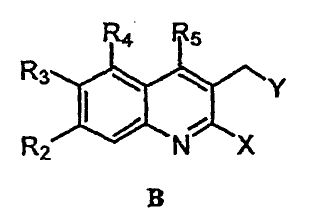 in der R₂, R₃, R₄ und R₅ die in Anspruch 1 angegebene Bedeutung haben, X ein Chlor-, Brom- oder Iodatom darstellt und Y entweder ein Bromatom oder eine Hydroxylfunktion darstellt,
*N*-alkyliert, um die Verbindung der allgemeinen Formel C 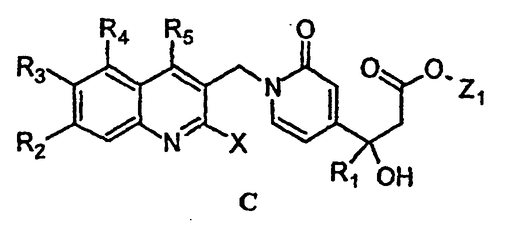 in der R₁, R₂, R₃, R₄, R₅, X und Z₁ die in Anspruch 1 angegebene Bedeutung haben, zu erhalten,
dann die Verbindung der allgemeinen Formel C cyclisiert, um die Verbindung der allgemeinen Formel (I) gemäß Anspruch 1 zu erhalten, in der R₁₀ einen Carbalkoxyrest darstellt.

7. Verfahren zur Herstellung einer Verbindung der allgemeinen Formel (I), **dadurch gekennzeichnet, dass** man
ein Pyridinon der allgemeinen Formel D 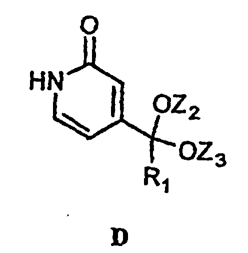 in der R₁ die in Anspruch 1 angegebene Bedeutung hat und die Gruppen Z₂ und Z₃ unabhängig voneinander ein verzweigtes oder unverzweigtes Alkyl mit 1 bis 6 Kohlenstoffatomen darstellen oder Z₂ und Z₃ zusammen eine gesättigte Kohlenwasserstoffkette mit 2 bis 4 Kohlenstoffen bilden,
mit einem Chinolin der allgemeinen Formel B gemäß Anspruch 6 *N*-alkyliert, um die Verbindung der allgemeinen Formel E 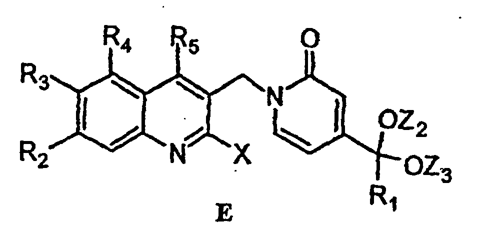 zu erhalten, in der R₁, R₂, R₃, R₄, R₅, X, Z, und Z₃ die in Anspruch 1 angegebene Bedeutung haben,
dann die Verbindung der allgemeinen Formel E cyclisiert, um die Verbindung der allgemeinen Formel F 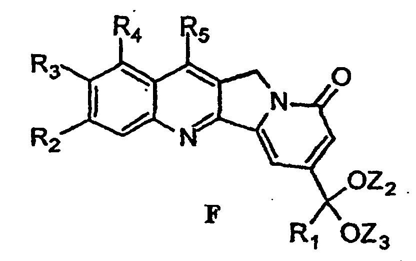 zu erhalten, in der R₁, R₂, R₃, R₄, R₅, Z₂ und Z₃ die in Anspruch 1 angegebene Bedeutung haben,
und dann die geschützte Carbonylfunktion der Verbindung der allgemeinen Formel F freisetzt, um die Verbindung der allgemeinen Formel G zu erhalten 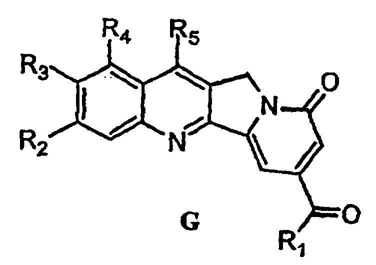 in der R₁, R₂, R₃, R₄ und R₅ die in Anspruch 1 angegebene Bedeutung haben,
und dann die Carbonylfunktion der Verbindung der allgemeinen Formel G mit einem epoxidierendes Mittel behandelt, um die Verbindung der allgemeinen Formel H zu erhalten 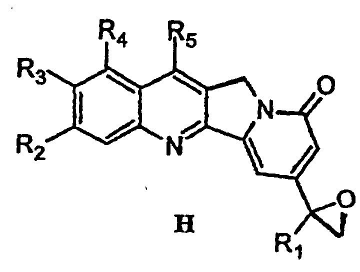 in der R₁, R₂, R₃, R₄ und R₅ die in Anspruch 1 angegebene Bedeutung haben,
und dann das Epoxid der Verbindung der allgemeinen Formel H mit einem nitrilierenden Mittel behandelt, um die allgemeine Verbindung der allgemeinen Formel (I) gemäß Anspruch 1 zu erhalten, in der R₁₀ einen Cyanorest darstellt.

8. Verfahren zur Herstellung einer Verbindung der allgemeinen Formel (I) nach einem der Ansprüche 1 bis 5, **dadurch gekennzeichnet, dass** die Carbonylfunktion einer Verbindung der allgemeinen Formel G gemäß Anspruch 7 mit einem geeigneten alkylierenden Mittel behandelt wird, um die Verbindung der entsprechenden allgemeinen Formel (I) zu ergeben.

9. Verfahren zur Herstellung einer Verbindung der allgemeinen Formel (I) nach einem der Ansprüche 1 bis 5, **dadurch gekennzeichnet, dass** die Esterfunktion einer Verbindung der allgemeinen Formel (I), in der R₁₀ einen Carbalkoxyrest darstellt, verseift wird, um eine Verbindung der allgemeinen Formel (I) zu ergeben, in der R₁₀ einen Carboxyrest darstellt.

10. Verfahren zur Herstellung der Verbindungen der allgemeinen Formel (I) nach einem der Ansprüche 1 bis 5, **dadurch gekennzeichnet, dass** die Säurefunktion einer Verbindung der allgemeinen Formel (I), in der R₁₀ einen Carboxyrest darstellt, verestert wird, um eine Verbindung der allgemeinen Formel (I) zu ergeben, in der R₁₀ einen Carbalkoxyrest darstellt.

11. Verfahren zur Herstellung der Verbindungen der allgemeinen Formel (I) nach einem der Ansprüche 1 bis 5, **dadurch gekennzeichnet, dass** die Esterfunktion einer Verbindung der allgemeinen Formel (I), in der R₁₀ einen Carbalkoxyrest darstellt, umgeestert wird, um eine Verbindung der allgemeinen Formel (I) zu ergeben, in der R₁₀ einen anderen Carbalkoxyrest darstellt.

12. Verfahren zur Herstellung der Verbindungen der allgemeinen Formel (I), **dadurch gekennzeichnet, dass** die Nitrilfunktion einer Verbindung der allgemeinen Formel(I), in der R₁₀ einen Cyanorest darstellt, einer dipolaren Addition mit einem Nitrid unterworfen wird, um eine Verbindung der allgemeinen Formel (I) zu ergeben, in der R₁₀ einen 1,2,3,4-Tetrazol-5-yl-Rest darstellt.

13. Verbindungen der allgemeinen Formel A 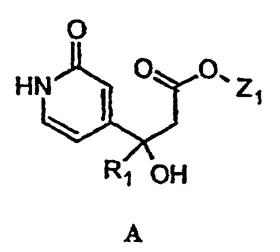 in der R₁ einen Alkyl-, Alkenyl-, Alkinyl-, Halogenalkyl-, Alkoxyalkyl- oder Alkylthioalkylrest darstellt und die Gruppe Z₁ einen Alkylrest darstellt, in Form von neuen Industrieprodukten,
wobei
jede der in den Definitionen der oben genannten Reste auftretenden Alkylgruppen verzweigt oder unverzweigt ist und 1 bis 6 Kohlenstoffatome besitzt und
jede der in den Definitionen der oben genannten Reste auftretenden Alkenyloder Alkinylgruppen verzweigt oder unverzweigt ist und 2 bis 6 Kohlenstoffatome aufweist.

14. Verfahren zur Herstellung der Verbindungen der allgemeinen Formel A nach Anspruch 13, **dadurch gekennzeichnet, dass** man von einem 2-Alkoxypyridin der allgemeinen Formel J 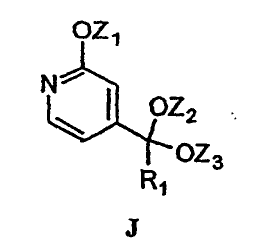 in der R₁ einen Alkyl-, Alkenyl-, Alkinyl-, Halogenalkyl-, Alkoxyalkyl- oder Alkylthioalkylrest darstellt, die Gruppe Z₁ einen Alkylrest darstellt und die Gruppen Z₂ und Z₃ unabhängig voneinander einen Alkylrest darstellen oder Z₂ und Z₃ zusammen eine gesättigte Kohlenwasserstoffkette mit 2 bis 4 Kohlenstoffen bilden, die Schutzgruppe entfernt, um ein Pyridinon der allgemeinen Formel K zu erhalten 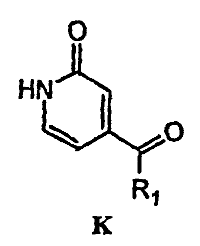 in der R₁ die in Anspruch 1 angegebene Bedeutung hat;
dann die Verbindung der allgemeinen Formel K mit einem funktionalisierten alkylierenden Mittel behandelt, um eine Verbindung der allgemeinen Formel A zu erhalten, in der R₁ und Z₁ die in Anspruch 13 angegebene Bedeutung haben;
wobei
jede der in den Definitionen der oben genannten Reste auftretenden Alkylgruppen verzweigt oder unverzweigt ist und 1 bis 6 Kohlenstoffatome aufweist und
jede der in den Definitionen der oben genannten Reste auftretenden Alkenyloder Alkinylgruppen verzweigt oder unverzweigt ist und 2 bis 6 Kohlenstoffatome aufweist.

15. Verbindungen der allgemeinen Formel D 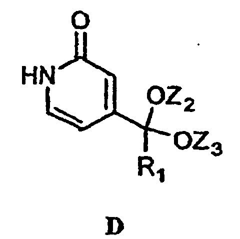 in der R₁ einen Alkyl-, Alkenyl-, Alkinyl-, Halogenalkyl-, Alkoxyalkyl- oder Alkylthioalkylrest darstellt und die Gruppen Z₂ und Z₃ unabhängig voneinander einen Alkylrest darstellen oder Z₂ und Z₃ zusammen eine gesättigte Kohlenwasserstoffkette mit 2 bis 4 Kohlenstoffen bilden, in Form von neuen Industrieprodukten,
wobei
jede der in den Definitionen der oben genannten Reste auftretenden Alkylgruppen verzweigt oder unverzweigt ist und 1 bis 6 Kohlenstoffatome aufweist und
jede der in den Definitionen der oben genannten Reste auftretenden Alkenyloder Alkinylgruppen verzweigt oder unverzweigt ist und 2 bis 6 Kohlenstoffatome aufweist.

16. Verfahren zur Herstellung der Verbindungen der allgemeinen Formel D nach Anspruch 15, **dadurch gekennzeichnet, dass** man die Ketonfunktion in der Verbindung der allgemeinen Formel K gemäß Anspruch 14 schützt, um eine Verbindung der allgemeinen Formel D zu erhalten, in der R₁, Z₂ und Z₃ die in Anspruch 15 angegebene Bedeutung haben.

17. Verbindungen der allgemeinen Formel K 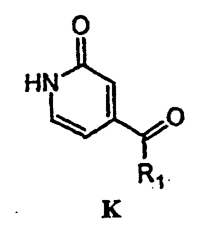 in der R₁ einen Alkyl-, Alkenyl-, Alkinyl-, Halogenalkyl-, Alkoxyalkyl- oder Alkythioalkylrest darstellt, in Form von neuen Industrieprodukten,
wobei
jede der in den Definitionen der oben genannten Reste auftretenden Alkylgruppen verzweigt oder unverzweigt ist und 1 bis 6 Kohlenstoffatome besitzt und
jede der in den Definitionen der oben genannten Reste auftretenden Alkenyloder Alkinylgruppen verzweigt oder unverzweigt ist und 2 bis 6 Kohlenstoffatome besitzt.

18. Verbindung nach einem der Ansprüche 1 bis 5 oder ein pharmazeutisch verträgliches Salz dieser Verbindung in Form eines Arzneimittels.

19. Pharmazeutische Verbindung, die als Wirkstoff mindestens eine der Verbindungen nach einem der Ansprüche 1 bis 5 enthält.

20. Verwendung einer Verbindung nach einem der Ansprüche 1 bis 5 für die Herstellung von Arzneimitteln zur Hemmung der Topoisomerasen vom Typ I oder der Topoisomerasen vom Typ II oder beider Typen von Topoisomerasen gleichzeitig.

21. Verwendung einer Verbindung nach einem der Ansprüche 1 bis 5 für die Herstellung von Arzneimitteln zur Behandlung von Tumoren.

22. Verwendung einer Verbindung nach einem der Ansprüche 1 bis 5 für die Herstellung von antiviralen Arzneimitteln.

23. Verwendung einer Verbindung nach einem der Ansprüche 1 bis 5 für die Herstellung von antiparasitären Arzneimitteln.

## Claims

1. A compound **characterized in that** said compound is of formula (I), 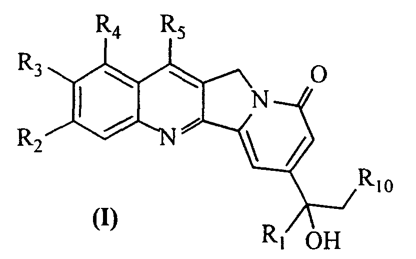 in racemic or enantiomeric form or any combination of these forms, in which
R₁ represents a lower alkyl, lower alkenyl, lower alkynyl, lower haloalkyl, lower alkoxyalkyl or lower alkylthioalkyl radical;
R₂, R₃ and R₄ represent, independently, an H, hydroxy, lower alkoxy, arylalkoxy, halo, lower haloalkyl, lower alkyl, lower alkenyl, cyano, lower cyanoalkyl, nitro, lower nitroalkyl, amido, lower amidoalkyl, (CH₂)ₘNR₆R₇, (CH₂)ₘOR₆, (CH₂)ₘSR₆, (CH₂)ₘCO₂R₆, (CH₂)ₘNR₆C(O)R₈, (CH₂)ₘC(O)R₈, (CH₂)ₘOC(O)R₈, O(CH₂)ₘNR₆R₇, OC(O)NR₆R₇, OC(O)(CH₂)ₘCO₂R₆, aryl or lower arylalkyl radical substituted (i.e., substituted one to four times on the aryl group) or non-substituted, in which the substituent is a lower alkyl, halo, nitro, amino, lower alkylamino, lower haloalkyl, lower hydroxyalkyl, lower alkoxy, or lower alkoxyalkyl) or R₂ and R₃, or R₃ and R₄, or R₄ and R₅, independently form together a chain with 3 or 4 links, in which the elements of the chain are selected from the group constituted by CH, CH₂, O, S, N or NR₉;
R₅ represents an H, halo, lower haloalkyl, lower alkyl, lower alkoxy, lower alkoxyalkyl, lower alkylthioalkyl, cycloalkyl, lower cycloalkylalkyl, cyano, cyanoalkyl, lower alkanesulphonylalkyl, lower hydroxyalkyl, nitro, (CH₂)ₘC(O)R₈, (CH₂)ₘNR₆C(O)R₈, (CH₂)ₘNR₆R₇, (CH₂)ₘN(CH₃)(CH₂)ₙNR₆R₇, (CH₂)ₘOC(O)R₈, (CH₂)ₘOC(O)NR₆R₇, aryl or lower arylalkyl radical substituted (i.e. one to four times on the aryl group) or non-substituted, in which the substituent is a lower alkyl, halo, nitro, amino, lower alkylamino, lower haloalkyl, lower hydroxyalkyl, lower alkoxy or lower alkoxyalkyl;
R₆ and R₇ represent, independently, H, a lower alkyl, lower hydroxyalkyl, lower alkylaminoalkyl, lower aminoalkyl, cycloalkyl, lower cycloalkylalkyl, lower alkenyl, lower alkoxyalkyl, lower haloalkyl, or aryl or lower arylalkyl radical substituted (i.e., one to four times on the aryl group) or non-substituted, in which the substituent is a lower alkyl, halo, nitro, amino, lower alkylamino, lower haloalkyl, lower hydroxyalkyl, lower alkoxy, or lower alkoxyalkyl, or, when the chains R₆ and R₇ are attached to the same nitrogen atom, R₆ and R₇ optionally together form an aromatic or non-aromatic heteroring, for example a heteroring of morpholine, piperazine or piperidine type, said heteroring being optionally substituted by one or more groups chosen from the lower alkyl, substituted or non-substituted aryl, substituted or non-substituted arylalkyl, halo, nitro, amino, lower alkylamino, lower haloalkyl, lower hydroxyalkyl, lower alkoxy or lower alkoxyalkyl radicals;
R₈ represents an H, lower alkyl, lower hydroxyalkyl, amino, lower alkylamino, lower alkylaminoalkyl, lower aminoalkyl, cycloalkyl, lower cycloalkylalkyl, lower alkenyl, lower alkoxy, lower alkoxyalkyl, lower haloalkyl, or aryl or lower arylalkyl radical substituted (i.e., one to four times on the aryl group) or non-substituted, in which the substituent is a lower alkyl, halo, nitro, amino, lower alkylamino, lower haloalkyl, lower hydroxyalkyl, lower alkoxy, or lower alkoxyalkyl radical;
R₉ represents an H, lower alkyl, lower haloalkyl, aryl or arylalkyl radical, the aryl or arylalkyl group optionally being able to be substituted on the aromatic ring by one or more groups chosen from the lower alkyl, halo, nitro, amino, lower alkylamino, lower haloalkyl, lower hydroxyalkyl, lower alkoxy, or lower alkoxyalkyl radicals;
R₁₀ represents a cyano, C(O)OR₁₁, 1*H*-1,2,3,4-tetrazo-5-yl or 1-alkyl-1,2,3,4-tetrazo-5-yl radical;
R₁₁ represents an H, lower alkyl, lower haloalkyl, lower hydroxyalkyl, alkylcarbonyloxyalkyl, (CH₂)ₚNR₆R₇, aryl, arylalkyl or aryl radical, the aryl or arylalkyl group optionally being able to be substituted on the aromatic ring by one or more groups chosen from the lower alkyl, halo, nitro, amino, lower alkylamino, lower haloalkyl, lower hydroxyalkyl, lower alkoxy, or lower alkoxyalkyl radical;
m is an integer comprised between 0 and 6;
n is an integer comprised between 1 and 4;
p is an integer comprised between 2 and 6;
or a pharmaceutically acceptable salt of the latter.

2. A compound according to claim 1, **characterized in that**
R₁ represents a lower alkyl radical;
R₂ represents a hydrogen or halogen atom;
R₃ represents a hydrogen or halogen atom, or a lower alkyl or OR₆ radical in which R₆ represents H, a lower alkyl or lower arylalkyl radical;
R₄ represents a hydrogen atom;
R₅ represents a hydrogen atom or a lower alkyl radical;
R₁₀ represents a cyano, C(O)OR₁₁ or 1H-1,2,3,4-tetrazo-5-yl radical;
R₁₁ represents an H, lower alkyl, lower haloalkyl, lower hydroxyalkyl, alkylcarbonyloxyalkyl, (CH₂)ₚNR₆R₇, aryl, arylalkyl or aryl radical, the aryl or arylalkyl group optionally being able to be substituted on the aromatic ring by one or more groups chosen from the lower alkyl, halo, nitro, amino, lower alkylamino, lower haloalkyl, lower hydroxyalkyl, lower alkoxy, or lower alkoxyalkyl radical;
or a pharmaceutically acceptable salt of the latter.

3. A compound according to claim 2, **characterized in that**
R₁ represents an ethyl group;
R₂ represents a hydrogen, chlorine or fluorine atom;
R₃ represents a hydrogen, chlorine or fluorine atom, or a methyl, methoxy or benzyloxy radical;
R₄ represents a hydrogen atom;
R₅ represents a hydrogen atom or a lower alkyl radical;
R₁₀ represents a cyano, C(O)OR₁₁ or 1H-1,2,3,4-tetrazo-5-yl radical;
R₁₁ represents an H, lower alkyl, lower haloalkyl, lower hydroxyalkyl, alkylcarbonyloxyalkyl, (CH₂)ₚNR₆R₇, aryl, arylalkyl or aryl radical, the aryl or arylalkyl group optionally being able to be substituted on the aromatic ring by one or more groups chosen from the lower alkyl, halo, nitro, amino, lower alkylamino, lower haloalkyl, lower hydroxyalkyl, lower alkoxy, or lower alkoxyalkyl radical;
or a pharmaceutically acceptable salt of the latter.

4. A compound according to one of claims 1 to 3, **characterized in that** said compound is chosen from the compounds corresponding to the following formulae:
- *tert*-butyl 3-(9-benzyloxy-10-fluoro-4-oxo-4,6-dihydroindolizino [1,2-*b*]quinoline-2-yl)-3-hydroxypentanoate;
- *tert*-butyl 3-(10-fluoro-9-methoxy-4-oxo-4,6-dihydroindolizino [1,2-*b*] quinoline-2-yl)-3 -hydroxypentanoate;
- *tert*-butyl 3-hydroxy-3-(7-methyl-4-oxo-4,6-dihydroindolizino [1,2-*b*] quinoline-2-yl)pentanoate;
- 3-hydroxy-3-(4-oxo-4,6-dihydroindolizino [1,2-*b*] quinoline-2-yl)pentanitrile;
- *tert*-butyl 3-hydroxy-3-(4-oxo-4,6-dihydroindolizino [1,2-*b*] quinoline-2-yl)pentanoate;
- 3-hydroxy-3-(4-oxo-4,6-dihydroindolizino [1,2-*b*] quinoline-2-yl)pentanoic acid;
- 3-(9-benzyloxy-10-fluoro-4-oxo-4,6-dihydroindolizino [1,2-*b*]quinoline-2-yl)-3-hydroxypentanoic acid;
- 3-(10-fluoro-9-methoxy-4-oxo-4,6-dihydro-indolizino [1,2-*b*] quinoline-2-yl)-3-hydroxypentanoic acid;
- 3-hydroxy-3-(7-methyl-4-oxo-4,6-dihydroindolizino [1,2-*b*] quinoline-2-yl)pentanoic acid;
- 3-(9-benzyloxy-4-oxo-4,6-dihydroindolizino [1,2-*b*] quinoline-2-yl)-3-hydroxypentanoic acid;
- 3-(10-chloro-9-methyl-4-oxo-4,6-dihydro-indolizino [1,2-*b*] quinoline-2-yl)-3-hydroxypentanoic acid;
- methyl 3-hydroxy-3-(4-oxo-4,6-dihydroindolizino [1,2-*b*] quinoline-2-yl)pentanoate;
- *tert*-butylcarbonyloxy-methyl 3-hydroxy-3-(4-oxo-4,6-dihydroindolizino [1,2-*b*] quinoline-2-yl)pentanoate;
- 2-[1-hydroxy-1-(1H-1,2,3,4-tetrazo-5-ylmethyl)propyl]-4,6-dihydroindolizino [1,2-*b*] quinoline-4-one;
or a pharmaceutically acceptable salt of the latter.

5. A compound according to one of claims 1 to 4, **characterized in that** it is 3-hydroxy-3-(4-oxo-4,6-dihydroindolizino [1,2-*b*] quinoline-2-yl)pentanoic acid or a pharmaceutically acceptable salt of the latter.

6. A preparation process for the compounds of general formula (I) according to any one of claims 1 to 5, **characterized in that**
- a pyridinone of general formula A 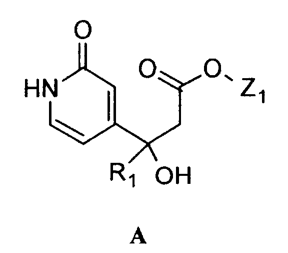 in which R₁ has the meaning indicated in claim 1 and the group Z₁ represents a lower alkyl radical, is *N*-alkylated with a quinoline of general formula B 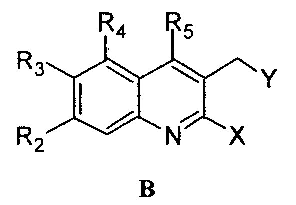 in which R₂, R₃, R₄ and R₅ have the meaning indicated in claim 1, X represents a chlorine, bromine or iodine atom and Y represents either a bromine atom, or a hydroxyl function, in order to produce the compound of general formula C 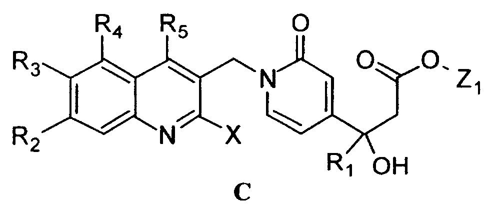 in which R₁, R₂, R₃, R₄, R₅, X, and Z₁ have the meaning indicated in claim 1,
- then the compound of general formula C is cyclized in order to obtain the compound of general formula (I) as defined in claim 1, in which R₁₀ represents a carbalkoxy radical.

7. A preparation process for a compound of general formula (I) **characterized in that**
- a pyridinone of general formula D 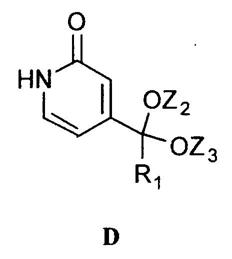 in which R₁ has the meaning indicated in claim 1 and the Z₂ and Z₃ groups represent, independently, a lower alkyl or Z₂ and Z₃ form together a saturated hydrocarbonated chain of 2 to 4 carbons, is *N*-alkylated with a quinoline of general formula B as defined in claim 6 in order to produce the compound of general formula E 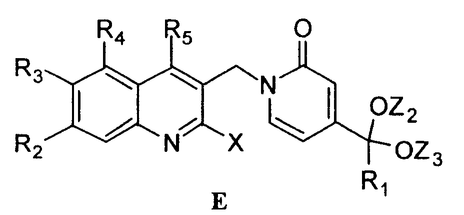 in which R₁, R₂, R₃, R₄, R₅, X, Z₂ and Z₃ have the meaning indicated in claim 1,
- then the compound of general formula E is cyclized in order to obtain the compound of general formula F 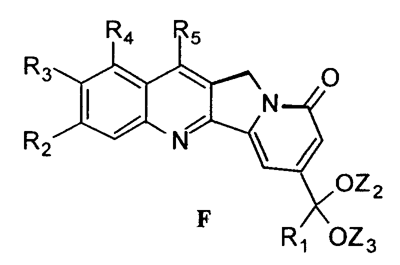 in which R₁, R₂, R₃, R₄, R₅, Z₂ and Z₃ have the meaning indicated in claim 1,
- then the protected carbonyl function of the compounds of general formula F is released in order to produce the compound of general formula G 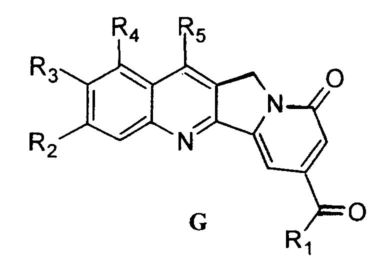 in which R₁, R₂, R₃, R₄, and R₅ have the meaning indicated in claim 1,
- then the carbonyl function of the compound of general formula G is treated with an epoxydizing agent in order to produce the compound of general formula H 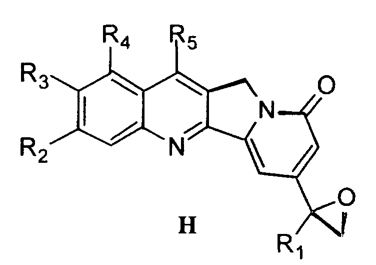 in which R₁, R₂, R₃, R₄, and R₅ have the meaning indicated in claim 1,
then the epoxide of the compound of general formula H is treated with a nitrilating agent in order to produce the compound of general formula (I) as defined in claim 1, in which R₁₀ represents a cyano radical.

8. A preparation process for a compound of general formula (I) according to one of claims 1 to 5, **characterized in that** the carbonyl function of a compound of general formula G as defined in claim 7 is treated with an appropriate alkylating agent in order to produce the corresponding compound of general formula (I).

9. A preparation process for a compound of general formula (I) according to one of claims 1 to 5, **characterized in that** the ester function of a compound of general formula (I) in which R₁₀ represents a carbalkoxy radical is saponified in order to produce a compound of general formula (I) in which R₁₀ represents a carboxy radical.

10. A preparation process for the compounds of general formula (I) according to one of claims 1 to 5, **characterized in that** the acid function of a compound of general formula (I) in which R₁₀ represents a carboxy radical is esterified in order to produce a compound of general formula (I) in which R₁₀ represents a carbalkoxy radical.

11. A preparation process for the compounds of general formula (I) according to one of claims 1 to 5 **characterized in that** the ester function of a compound of general formula (I) in which R₁₀ represents a carbalkoxy radical is transesterified in order to produce a compound of general formula (I) in which R₁₀ represents another carbalkoxy radical.

12. A preparation process for the compounds of general formula (I) **characterized in that** the nitrile function of a compound of general formula (I) in which R₁₀ represents a cyano radical undergoes a dipolar addition with a nitride in order to produce a compound of general formula (I) in which R₁₀ represents a 1,2,3,4-tetrazole-5-yl radical.

13. As new industrial products, the compounds of general formula A 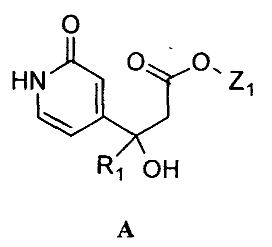 in which R₁ represents a lower alkyl, lower alkenyl, lower alkynyl, lower haloalkyl, lower alkoxyalkyl or lower alkylthioalkyl radical, and the Z₁ group represents a lower alkyl radical.

14. A preparation process for the compounds of general formula A according to claim 13, **characterized in that** a 2-alkoxypyridine of general formula J 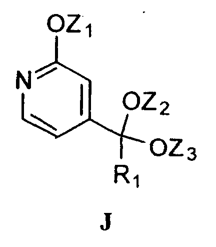 in which R₁ represents a lower alkyl, lower alkenyl, lower alkynyl, lower haloalkyl, lower alkoxyalkyl or lower alkylthioalkyl radical, the Z₁ group represents a lower alkyl radical and the Z₂ and Z₃ groups represent, independently, a lower alkyl radical or Z₂ and Z₃ form together a saturated hydrocarbon chain of 2 to 4 carbons, is deprotected in order to produce the pyridinone of general formula K 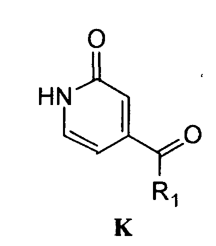 in which R₁ has the meaning indicated in claim 1;
then the compound of general formula K is treated with a functionalized alkylating agent in order to obtain a compound of general formula A in which R₁ and Z₁ have the meaning indicated in claim 13.

15. As new industrial products, the compounds of general formula D 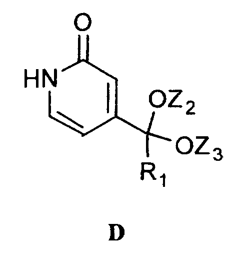 in which R₁ represents a lower alkyl, lower alkenyl, lower alkynyl, lower haloalkyl, lower alkoxyalkyl or lower alkylthioalkyl radical, and the Z₂ and Z₃ groups represent, independently, a lower alkyl radical or Z₂ and Z₃ form together a saturated hydrocarbon chain of 2 to 4 carbons.

16. A preparation process for the compounds of general formula D according to claim 15, **characterized in that** the ketonic function in the compound of general formula K as defined in claim 14 is protected in order to obtain a compound of general formula D in which R₁, Z₂ and Z₃ have the meaning indicated in claim 15.

17. As new industrial products, the compounds of general formula K 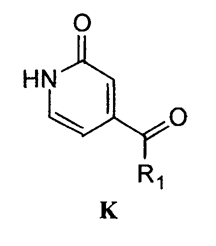 in which R₁ represents a lower alkyl, lower alkenyl, lower alkynyl, lower haloalkyl, lower alkoxyalkyl or lower alkylthioalkyl radical.

18. As a medicament, a compound according to any one of claims 1 to 5, or a pharmaceutically acceptable salt of the latter.

19. Pharmaceutical composition containing, as active ingredient, at least one of the compounds according to any one of claims 1 to 5.

20. Use of a compound according to any one of claims 1 to 5 for the preparation of medicaments intended to inhibit the topoisomerases of type I, or the topoisomerases of type II, or, simultaneously, both types of topoisomerase.

21. Use of a compound according to any one of claims 1 to 5 for the preparation of antitumoural medicaments.

22. Use of a compound according to any one of claims 1 to 5 for the preparation of antiviral medicaments.

23. Use of a compound according to any one of claims 1 to 5 for the preparation of antiparasitic medicaments.
